# FASCICULE DE BREVET EUROPEEN

(11) **EP 1 161 267 B1**
(45) Date de publication et mention de la délivrance du brevet: **03.09.2003**
(21) Numéro de dépôt: 00910967.9
(22) Date de dépôt: 16.03.2000
(51) Int. Cl.: A61L 2/14

(54) **PROCEDE ET DISPOSITIF DE STERILISATION PAR PLASMA**
VERFAHREN UND VORRICHTUNG ZUR PLASMASTERILISATION
METHOD AND DEVICE FOR STERILISATION BY PLASMA

(30) Priorité: 16.03.1999 FR 9903200
(43) Date de publication de la demande: 12.12.2001
(73) Titulaire: Absys, 92147 Clamart (FR)
(72) Inventeur: BOUSQUET, Séverine, F-91190 Gif sur Yvette (FR); DESTREZ, Philippe, F-92190 Meudon (FR); JAFFREZIC, Marie-Pierre, F-78140 Velizy (FR); PERRUCHOT, François, F-92130 Issy les Moulineaux (FR)
(74) Mandataire: David, Alain
(86) Numéro de dépôt international: FR0000644
(87) Numéro de publication internationale: WO00054819

(56) Documents cités:
- EP-A- 0 474 137
- FR-A- 2 759 590
- US-A- 3 948 601
- US-A- 5 200 158
- US-A- 5 633 424

## Description

La présente invention se rapporte au domaine général de la stérilisation d'objets et de surfaces de toute nature et de toute forme et elle concerne plus particulièrement un procédé et différents dispositifs de stérilisation par plasma fonctionnant à température ambiante et à pression atmosphérique.

### Art antérieur

La stérilisation correspond à un niveau de qualité bien précis dans les milieux médicaux et agro-alimentaires. Dans le milieu médical, elle désigne une destruction de tous les micro-organismes quelle que soit leur nature. Selon la Pharmacopée Européenne, un objet peut être considéré comme stérile si la probabilité qu'un micro-organisme viable y soit présent est inférieure ou égale à 10⁻⁶. Le temps de stérilisation est le temps nécessaire pour stériliser un objet « normalement contaminé », c'est-à-dire contenant 10⁶ spores bactériennes. Ainsi, la stérilisation d'un objet correspond à une réduction de la population initiale de spores bactériennes présentes sur cet objet de 10⁶ spores à 10⁻⁶ spores soit une réduction logarithmique de 12 décades.

Actuellement, il existe de nombreux procédés permettant de rendre et de garder des objets stériles. L'article de Philip M Schneider paru dans le volume 77 du Tappi Journal de Janvier 1994 en pages 115 à 119 en donne un aperçu relativement exhaustif. Toutefois, Mr Schneider termine son propos sur le constat qu'il n'existe pas aujourd'hui une méthode de stérilisation idéale à basse température (c'est à dire à moins de 80°C), c'est à dire qui soit d'une grande efficacité, d'une action rapide et d'une forte pénétration, qui soit en outre non toxique, compatible avec de nombreux matériaux notamment les matériaux organiques et qui puisse être mise en oeuvre simplement avec de faibles coûts.

En outre, l'état stérile d'un objet doit être maintenu par un emballage spécifique qui doit être compatible avec la méthode de stérilisation employée (perméable à l'agent stérilisant) et empêcher la pénétration de micro-organismes pendant les phases de transport et de stockage, afin de garantir la stérilité de l'instrument lors d'une prochaine utilisation.

Les procédés de stérilisation actuels sont basés essentiellement sur l'effet de la chaleur ou sur l'action de gaz biocides.

L'autoclave, qui repose sur l'action de la chaleur humide à haute température (au moins 121°C), est le procédé le plus efficace et le moins cher à mettre en oeuvre, mais il ne permet pas la stérilisation de dispositifs thermosensibles de plus en plus présents, notamment dans le domaine médical.

Les procédés de stérilisation par les gaz (oxyde d'éthylène, fonnaldéhyde, peroxyde d'hydrogène) utilisent le caractère biocide d'un gaz placé dans une enceinte de stérilisation et permettent une stérilisation à basse température des dispositifs thermosensibles. Mais, ils présentent de nombreux inconvénients : la toxicité des gaz considérés qui impose des procédures d'utilisation et de contrôle complexes ; l'obligation, dans certains cas (avec des matériaux plastiques par exemple), de réaliser une phase de désorption du gaz toxique après la phase de stérilisation ; enfin la longueur du traitement qui nécessite souvent plusieurs heures. En outre, il convient de noter l'effet de destruction limité sur certaines spores bactériennes (comme les spores de *Bacillus stearothermophilus*).

Aussi, une amélioration connue de ces procédés de stérilisation par des gaz biocides consiste à effectuer le traitement à basse pression (quelques Torrs), ce qui permet de favoriser la diffusion du gaz ou la vaporisation d'un liquide biocide additionnel dans toute l'enceinte de stérilisation. De même, on peut optimiser ces procédés de stérilisation à basse pression en créant des cycles composés d'alternance entre des phases de diminution ou d'augmentation de la pression et des phases de traitement plasma comme le montre notamment la demande de brevet FR 2 759 590 déposée par la société SA Microondes Energie Systèmes.

On connaît également des procédés de stérilisation par plasma à basse pression qui permettent éventuellement de cumuler les effets stérilisants du gaz biocide à basse pression avec la formation, dans un plasma, à partir d'un mélange gazeux biocide tel que H₂O₂ ou un mélange gazeux non biocide (en général simplement O₂, H₂, H₂O, N₂ ou un gaz rare comme l'Argon) d'espèces réactives (création de radicaux O^{•} et OH^{•}, d'espèces ionisées et / ou excitées), la basse pression limitant par ailleurs la recombinaison des espèces instables. Les plasmas basse pression regroupent. dans la majorité des cas, des plasmas micro-ondes ou radiofréquences.

La stérilisation par plasma peut être très efficace dans l'espace où est créé le plasma (espace inter-électrodes), mais, outre que la zone de stérilisation est alors très petite (environ quelques cm de hauteur), les caractéristiques du plasma dépendent très fortement de la constante diélectrique, de la nature et de la taille de l'objet à stériliser. Dans ce cas, le plasma empêche un traitement réellement homogène sur toute la surface et a une action fortement corrosive sur les objets à stériliser.

Pour améliorer un tel procédé, il faut dissocier les zones de production du plasma et de traitement (la stérilisation est alors dite effectuée en post-décharge) afin d'éviter une trop grande interaction entre le plasma et l'objet à stériliser qui, de part sa nature, entraîne une perturbation des lignes de champ et une dégradation de la surface de l'objet lorsqu'il est placé dans l'espace inter-électrodes. Toutefois, on notera que cette séparation de la zone de production du plasma et de la zone de stérilisation est aisée dans un procédé basse pression car les durées de vie des radicaux produits par le plasma à ces pressions (d'environ 1 Torr) sont importantes, ce qui leur permet d'atteindre les objets à stériliser.

Les inconvénients des procédés de stérilisation par plasma à basse pression sont, malgré cela, encore nombreux et en définitive assez proches de ceux existants dans la seule stérilisation par les gaz : le coût élevé du système complet contenant une enceinte résistante au vide et un générateur de plasma ; la complexité des dispositifs mettant en oeuvre ces procédés qui limite les applications possibles ; l'augmentation du temps de traitement due aux procédures liées au traitement à basse pression (les temps nécessaires à la mise sous vide de l'enceinte, souvent de grand volume, puis au retour à la pression atmosphérique augmentent le temps de traitement) ; l'impossibilité de stériliser des objets humides ; et l'incompatibilité vis à vis de certains matériaux.

Aussi, un autre procédé connu consiste à effectuer un traitement par ozone à la pression atmosphérique à partir d'un dispositif approprié appelé « ozoneur ». Ce traitement s'apparente à une stérilisation par plasma en post-décharge dont le gaz vecteur, alors de type sec, contiendrait de l'oxygène. Toutefois, l'action biocide de l'ozone, qui est surtout utilisée pour la désinfection d'eau, de déchets, de gaz est assez limitée au niveau de la stérilisation. Pour obtenir de meilleures performances, il faut en général ajouter à l'ozone (O₃) un autre agent désinfectant (par exemple du ClO₂ afin de former du ClO₃ qui présente une action bactéricide en phase gazeuse). On peut également procéder à une humification du gaz ozoné en sortie de l'ozoneur ou bien simplement mouiller les objets à stériliser pour faciliter l'action biocide de ce gaz ozoné comme l'illustre le brevet US 5 120 512 (Masuda). De manière générale pour les procédés plasma à partir de gaz simples non biocides, la séparation entre la zone de production plasma et la zone de traitement limite l'efficacité du procédé car seules les espèces à moyenne et longue durée de vie sont encore actives au niveau de l'objet. Or, ces espèces étant moins réactives que celles à faible durée de vie, il est nécessaire d'augmenter leur concentration et le temps de traitement. Par exemple, il est connu que près de 3 heures de traitement sont nécessaires pour obtenir la stérilisation de spores de *Bacillus subtilis* en présence d'humidité pour une concentration en ozone de 1500 ppm. Un tel niveau de production impose l'utilisation de dispositifs complexes alors que la forte concentration en ozone augmente la dégradation irréversible des surfaces et matériaux des objets à stériliser. De plus, la production d'ozone en forte concentration rend nécessaire, du fait de la réglementation, le recours en sortie du système d'un dispositif destructeur d'ozone particulièrement efficace du type de ceux à catalyse ou par voie thermique.

Le document US-A-5 693 424 décrit la stérilisation par plasma dans une enceinte remplie de vapeur d'eau à pression sous-atmosphérique.

### Objet et définition de l'invention

L'objet de la présente invention est donc de proposer un dispositif de stérilisation par plasma mettant en oeuvre un procédé fonctionnant à basse température (de préférence à température ambiante), à une pression égale ou sensiblement égale à la pression atmosphérique, et dans un milieu ouvert ou fermé et qui en outre pallie les nombreux inconvénients précités de l'art antérieur.

Selon l'invention, il est proposé un procédé de stérilisation par plasma à pression égale ou sensiblement égale à la pression atmosphérique d'au moins un objet dans lequel :
a) on place le ou les objets à traiter dans une enceinte de traitement soumise sensiblement à la pression atmosphérique,
b) on introduit dans cette enceinte de traitement un ou plusieurs mélanges gazeux non-biocides dont l'un au moins contient de l'humidité,
c) on crée un plasma produisant des espèces chimiques à partir d'un des mélanges gazeux en générant au moyen d'une alimentation haute tension une décharge électrique entre une électrode à haute tension et une électrode de masse, ces deux électrodes étant placées dans cette enceinte de traitement,
d) on achemine les espèces chimiques du plasma en dehors de la zone inter-électrodes, vers la surface de ou des objets à traiter, et
e) on évacue les résidus gazeux résultant du traitement hors de l'enceinte de traitement.

Ainsi, avec ce fonctionnement à pression atmosphérique. il n'est pas nécessaire de recourir à des dispositifs complexes de fabrication de vide et à des gaz biocides.

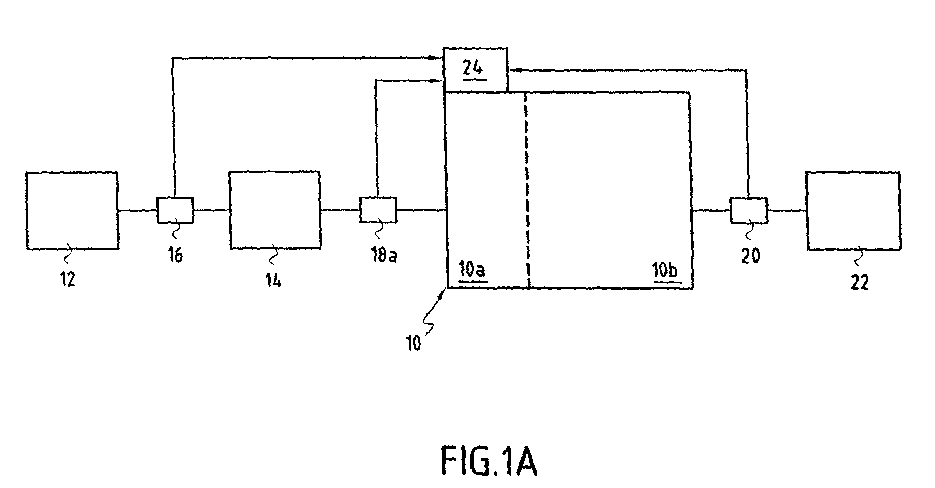

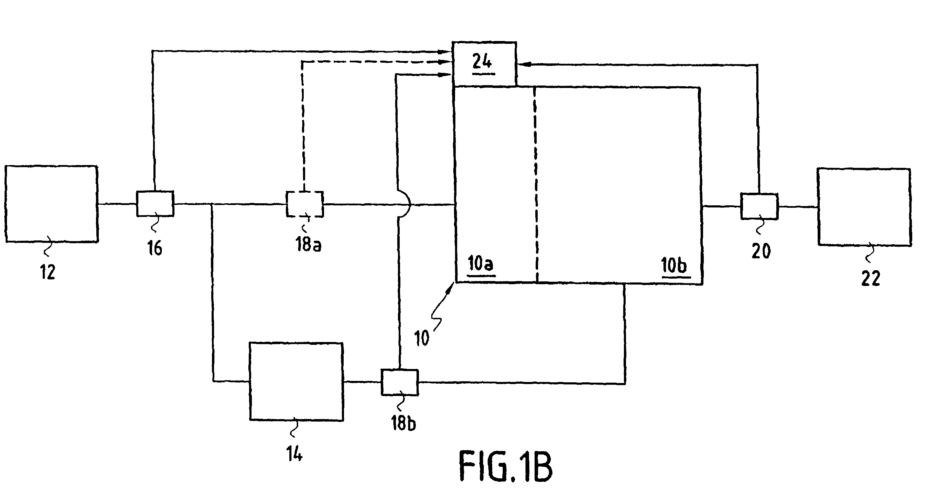

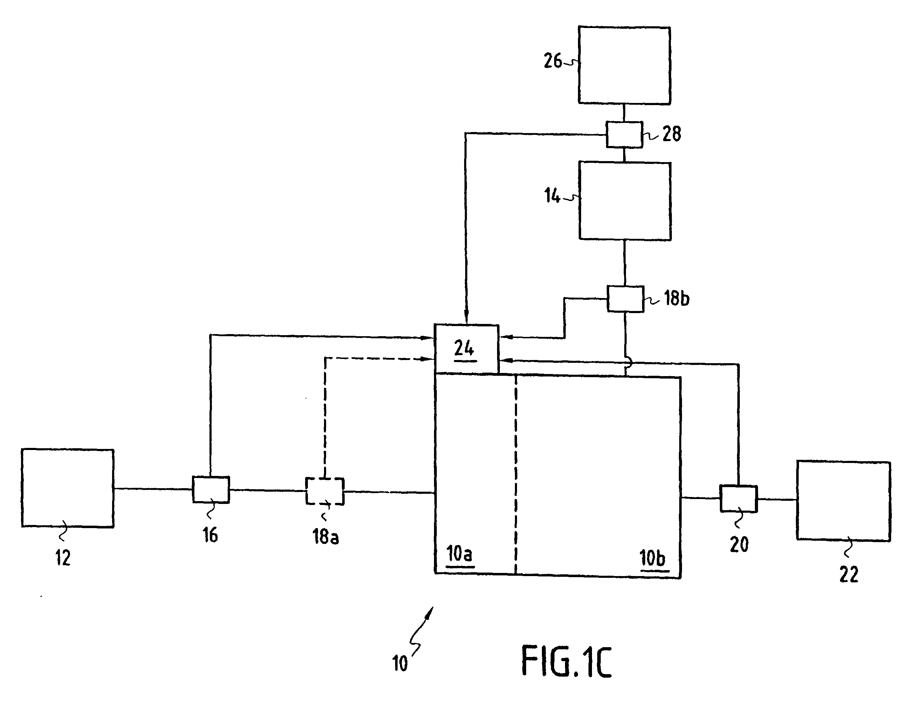

Selon le mode de réalisation envisagé, l'humidité est introduite soit directement au niveau du ou des objets à traiter (figures 1b et 1c) soit au niveau de la zone inter-électrodes (figure 1a).

Selon l'invention, le mélange gazeux contient au moins 10% d'oxygène et 10% d'azote. Il peut aussi être avantageusement constitué par de l'air ambiant.

De préférence, le taux d'humidité relative au niveau du ou des objets à traiter est compris entre 50% et 100%, avantageusement supérieur ou égal à 90%.

Selon le mode de réalisation retenu, l'étape b) d'introduction du ou des mélanges gazeux dans l'enceinte de traitement est effectuée de façon continue ou par intermittence et il est réalisé un contrôle du débit du ou des mélanges gazeux entrant dans l'enceinte de traitement. De même, l'étape c) de création du plasma peut être précédée par une étape de circulation forcée du ou des mélanges gazeux dans l'enceinte de traitement.

L'étape d) d'acheminement des espèces chimiques du plasma vers la surface à traiter est obtenue soit par l'utilisation du vent électrique créé par la décharge entre les deux électrodes soit par la création d'un écoulement forcé dans l'enceinte de traitement.

L'invention concerne aussi le dispositif mettant en oeuvre ce procédé, lequel comporte :
- une première source de gaz contenant un mélange gazeux non-biocide,
- au moins une enceinte de traitement soumise à la pression atmosphérique comportant au moins une zone de stérilisation dans laquelle sont placés le ou les objets à traiter, cette enceinte comportant en outre dans au moins une zone de production du plasma distincte de la zone de stérilisation au moins deux électrodes reliées à une alimentation haute tension pour créer un plasma produisant des espèces chimiques en générant une décharge électrique entre ces électrodes à partir du mélange gazeux introduit dans la zone de production du plasma. les espèces chimiques du plasma étant acheminées en dehors de la zone de production du plasma vers la surface de ou des objets à traiter et les résidus gazeux résultant du traitement étant évacués vers un système de récupération par un orifice de sortie de cette enceinte, et
- une chambre d'humidification reliée en sortie d'une seconde source de gaz pour maintenir un taux d'humidité déterminé au niveau du ou des objets à traiter.

Dans un mode de réalisation préférentiel, les première et seconde sources de gaz forment une source unique de gaz. La zone de production du plasma est alors soit reliée à cette source unique de gaz au travers de la chambre d'humidification soit reliée directement à cette source unique de gaz, la zone de stérilisation étant reliée à cette source unique de gaz au travers de la chambre d'humidification. En alternative, la zone de stérilisation peut aussi être reliée à la seconde source de gaz au travers de la chambre d'humidification, la zone de production du plasma étant alors reliée directement à la première source de gaz.

Le dispositif peut comporter des premier et second capteurs d'humidité relative placés respectivement en entrée de la zone de production du plasma et de la zone de stérilisation. Le second capteur d'humidité s'avère particulièrement utile lorsque la première source de gaz alimente directement l'enceinte de traitement (donc sans passer au travers de la chambre d'humidification) et ne fournit pas un gaz à humidité constante.

Les électrodes sont constituées par au moins une électrode à fort rayon de courbure et une électrode à faible rayon de courbure, l'une étant une électrode à haute tension et l'autre étant une électrode de masse. De préférence, l'une ou l'autre des deux électrodes, ou les deux, sont recouvertes d'un revêtement diélectrique.

Selon l'application envisagée, l'électrode à faible rayon de courbure est une électrode métallique pouvant présenter l'une des formes suivantes : fil, pointes ou fil comportant des pointes et l'électrode à fort rayon de courbure est une électrode métallique pouvant présenter l'une des formes suivantes : fil, plan, cylindre plein ou grille. Dans un exemple de réalisation particulièrement adaptée à la stérilisation de fins canaux, l'électrode à haute tension est constituée par un fil et l'électrode de masse est constituée par un cylindre grillagé entourant ce fil.

De même, les électrodes peuvent être montées en un réseau d'électrodes parallèles, l'alimentation des électrodes à haute tension étant effectuée successivement ou de façon simultanée.

Avantageusement, les électrodes peuvent être à usage limité.

L'alimentation haute tension est assurée par un générateur basse fréquence délivrant une tension continue, en créneaux, alternative ou pulsée en temps.

Dans une configuration préférentielle envisagée pour le dispositif, celui-ci comporte plusieurs enceintes de traitement, chaque enceinte de traitement comportant au moins une zone de production de plasma reliée de façon fixe on non à au moins une zone de stérilisation, les zones de production de plasma étant reliées à une unité centrale commune contenant au moins la première source de gaz non-biocide, la chambre d'humidification, le système de récupération de résidus gazeux et l'alimentation haute tension.

Avantageusement, la zone de stérilisation présente une forme spécialement adaptée à ou aux objets à stériliser afin de limiter la production d'espèces chimiques nécessaires à la stérilisation et d'optimiser la vitesse d'écoulement et la concentration de ces espèces stérilisantes autour de l'objet. En outre, la zone de production du plasma peut être intégrée à l'objet à traiter dont elle en forme une partie.

Dans un mode de réalisation préférentiel, l'enceinte de traitement comporte un boîtier de forme standard et contenant la zone de production du plasma, la zone de stérilisation étant formée par un support amovible spécialement adapté à ou aux objets à traiter et logé dans ce boîtier.

Pour permettre un traitement homogène de toutes les parties d'un objet en diminuant le temps de propagation de la zone de production du plasma aux surfaces à stériliser, la zone de stérilisation peut comprendre des zones de propagation de faible section permettant d'accélérer les espèces chimiques provenant de la zone de production du plasma vers différentes parties du ou des objets à traiter.

Dans un mode de réalisation particulier, la zone de stérilisation est séparée de la zone de production du plasma et forme une enceinte indépendante.

Une ou plusieurs des enceintes de traitement ou de stérilisation peuvent constituer un emballage autonome réutilisable, en forme de mallette de transport, permettant de conserver l'état stérile après traitement ou un emballage perdu, en forme de sac souple, la zone de stérilisation pouvant être séparées en plusieurs zones individualisées après le traitement, par découpe et scellement concomitant de parties déterminées de ce sac. De préférence, tout ou partie du dispositif est placé dans une cage de Faraday.

Avantageusement, l'unité centrale commune comporte des moyens d'indication et de commande associés à chaque enceinte de stérilisation pour assurer un contrôle individuel de la stérilisation des objets qu'elle contient. Elle comporte également des moyens d'impression pour imprimer une étiquette sur laquelle seront imprimés, pour chaque enceinte de stérilisation reliée à cette unité centrale, un numéro d'identification spécifique à chaque enceinte ainsi que la date du traitement et les paramètres du cycle de stérilisation effectué. De même, l'enceinte de traitement ou de stérilisation est munie de préférence d'une étiquette électronique qui permet par l'intermédiaire d'un lecteur correspondant de l'unité centrale de déterminer automatiquement les valeurs de débit de consigne et le courant de régulation adaptés à ou aux objets à traiter et de calculer le temps nécessaire à la stérilisation de ces objets. Cette étiquette électronique peut comporter un capteur de vitesse pour mesurer la vitesse d'écoulement des espèces chimiques du plasma dans l'enceinte ainsi qu'un capteur de mesure chimique.

La grande souplesse du dispositif, reposant en partie sur la possibilité de choisir le nombre, la taille et la position des zones de production du plasma en fonction du volume et de la configuration des objets à traiter, permet son application à la stérilisation d'objets divers de toute forme et de toute nature, notamment métallique, composite ou thermosensible comme à la stérilisation des surfaces des emballages, des produits ou des matériels de production, à la décontamination des surfaces internes des systèmes d'air conditionné ou encore à la désinfection des zones de confinement ou de transfert. Son application est particulièrement intéressante dans les domaines médical, dentaire, agroalimentaire ou pharmaceutique.

### Brève description des dessins

D'autres caractéristiques et avantages de la présente invention ressortiront mieux de la description suivante effectuée à titre indicatif et non limitatif en regard des dessins annexés sur lesquels :
- la figure la est un schéma de principe d'un dispositif de stérilisation par plasma selon l'invention,
- les figures 1b et 1c sont des schémas de principe illustrant deux variantes de réalisation du dispositif de stérilisation par plasma selon l'invention,
- la figure 2 est un schéma de principe d'une enceinte de traitement mise en oeuvre dans le dispositif de la figure 1,
- la figure 3 illustre un premier exemple de réalisation du dispositif de stérilisation par plasma de la figure 1,
- la figure 4 montre un premier exemple de réalisation d'une enceinte de traitement conforme à la figure 2,
- la figure 5 montre un second exemple de réalisation d'une enceinte de traitement conforme à la figure 2,
- la figure 6 montre un troisième exemple de réalisation d'une enceinte de traitement conforme à la figure 2,
- la figure 6a est une vue éclatée de la figure 6 illustrant une configuration particulière d'électrodes,
- les figures 7a et 7b illustrent un quatrième exemple de réalisation d'une enceinte de traitement conforme à la figure 2, et
- la figure 8 illustre un second exemple de réalisation du dispositif de stérilisation par plasma de la figure 1.

### Description d'un mode préférentiel de réalisation

L'invention concerne un procédé de stérilisation ayant une efficacité sporicide sur des spores bactériennes considérées par la Pharmacopée Européenne comme les plus résistantes : *Bacillus subtilis* et *Bacillus stearothermophilus*. Ce procédé met en oeuvre un mélange gazeux contenant de l'oxygène et de l'azote à partir duquel est créé un plasma basse température dont les espèces chimiques ont une action stérilisante sur l'objet à traiter en présence d'humidité. L'objet à traiter est placé en dehors de l'espace où a lieu la décharge et le traitement se fait à la pression atmosphérique.

Le plasma est un gaz partiellement activé par une source électromagnétique d'énergie suffisante. Les espèces créées dans le plasma sont des espèces ionisées (molécules ou atomes), neutres (tels que les radicaux) ou excitées. Ces espèces gazeuses ont une réactivité accrue qui leur permet d'interagir avec les surfaces du ou des objets à stériliser et ainsi de détruire les micro-organismes présents sur ces surfaces. A pression atmosphérique pour un plasma crée à partir d'un gaz simple non-biocide, les espèces les plus réactives sont celles ayant la plus faible durée de vie, cette efficacité dépendant fortement de la distance entre la zone de création du plasma et l'objet.

Un premier schéma de principe d'un dispositif de stérilisation par plasma mettant en oeuvre le procédé selon l'invention est illustré à la figure 1a. Ce dispositif est organisé autour d'une enceinte de traitement 10 séparée en deux zones : une zone de production du plasma 10a à l'intérieur de laquelle, par une décharge entre deux électrodes, est créé un plasma et une zone de stérilisation 10b où est placé l'objet à traiter. La décharge est produite à partir d'un mélange gazeux non-biocide fourni par une source de gaz 12 et humidifié par passage au travers d'une chambre d'humidification 14. Cette enceinte de traitement peut être entièrement fermée (et alors étanche) ou seulement partiellement ouverte selon l'application envisagée.

Le mélange gazeux contient de l'oxygène et de l'azote et sa composition peut varier en fonction de la nature de l'objet à stériliser. Du taux d'oxygène contenu dans le mélange dépend l'agressivité du mélange vis à vis des matériaux constitutifs des objets à stériliser. Le mélange gazeux doit contenir au minimum 10% d'oxygène et 10% d'azote pour assurer un effet sporicide acceptable. Avantageusement, ce mélange peut être de l'air ambiant obtenu à partir d'un compresseur. Le taux d'humidité relative dans la zone de stérilisation, donc au niveau des objets à traiter, est compris entre 50% et 100%, avantageusement supérieur ou égal à 90%. Pour améliorer les temps de traitement, le dispositif de stérilisation peut être avantageusement contrôlé en température avec une température maximale n'excédant pas 80°C pour ne pas endommager certains types d'objets, comme les objets sensibles à la chaleur.

Le débit d'entrée du mélange gazeux dans l'enceinte de traitement 10 est ajusté en fonction de la taille et de la quantité des objets à stériliser, par un dispositif de contrôle 16 disposé en sortie de la source de gaz 12 et permettant un contrôle de sa vitesse d'écoulement et de sa concentration.

La décharge électrique crée en effet un plasma qui produit des espèces à durée de vie variable. Pour pouvoir bénéficier des espèces les plus actives (celles à faible durée de vie) tout en se plaçant en dehors de la décharge, il est nécessaire de s'assurer que le temps de propagation entre la zone de création du plasma et l'objet est minimum, donc que la vitesse de propagation entre ces deux éléments est maximum. Des expériences effectuées par les inventeurs ont cependant montré que, toute autre chose égales par ailleurs, l'efficacité du procédé proposé diminuait quand la vitesse locale du gaz au niveau de l'objet à stériliser augmentait. Il est donc aussi nécessaire de choisir un écoulement entre la zone de production du plasma et la zone de stérilisation qui assure un compromis entre une vitesse de propagation la plus élevée possible et une vitesse locale la plus faible possible.

La chambre d'humidification 14 permet d'humidifier jusqu'à saturer le mélange gazeux en humidité aux conditions normales de température et de pression. L'humidification directe de ce mélange permet de simplifier le procédé en limitant les alimentations en gaz et en simplifiant les écoulements. L'humidité relative du mélange gazeux humidifié (le gaz vecteur) est contrôlée avant son entrée dans l'enceinte de traitement 10 par un capteur approprié 18a placé en entrée de la zone de production du plasma 10a, la valeur de cette humidité relative conditionnant le temps de traitement nécessaire à la stérilisation. En effet, des essais effectués par les inventeurs ont pu montrer que plus l'humidité relative au niveau de l'objet est importante plus court est le temps de stérilisation (voir notamment l'exemple plus avant).

Le cycle de la stérilisation peut avoir lieu avec une circulation extérieure continue ou intermittente de gaz ou sans circulation extérieure de gaz. Il est cependant nécessaire qu'il existe une circulation de gaz (naturelle ou forcée) à l'intérieur de l'enceinte pour amener les espèces de la zone de production du plasma à la zone de stérilisation. Dans le premier cas, le débit du mélange gazeux est maintenu constant par le dispositif de contrôle 16 pendant tout le cycle de la stérilisation et les résidus (effluents) gazeux résultant de la décharge sont mesurés en sortie de l'enceinte de traitement 10 par un capteur de gaz 20 pour ne pas dépasser les concentrations limites fixées par la réglementation (ainsi dans le cas de l'ozone par exemple la valeur moyenne d'exposition admise sur un lieu de travail sur 8 heures est de 0,1 ppm selon la norme de l'Occupational Safety Health Administration), avant d'être si nécessaire évacués vers un système de récupération 22. Dans le deuxième cas, l'enceinte de traitement est remplie par le mélange gazeux à la pression atmosphérique puis isolée avant le cycle de stérilisation (le dispositif de contrôle 16 comportant à cet effet notamment une vanne d'isolation). le traitement ayant lieu dans un gaz non renouvelé. A la fin du cycle de stérilisation, l'atmosphère de l'enceinte de traitement 10 est renouvelée par passage du mélange gazeux avant d'être ouverte. Dans le troisième cas, le cycle de stérilisation peut éventuellement comporter une succession de phases intermittentes de circulation du mélange gazeux et de création du plasma.

Le capteur de gaz 20 placé en sortie de l'enceinte de traitement permet par l'intermédiaire de moyens de commande 24 disposés par exemple au niveau de cette enceinte de valider en temps réel le cycle de stérilisation et d'ajuster le temps de traitement en fonction du niveau de gaz dans l'enceinte et des conditions d'écoulement. Ces moyens de commande sont aussi avantageusement reliés au dispositif de contrôle 16 et au capteur d'humidité relative 18a pour assurer un contrôle entièrement automatique du cycle de stérilisation.

On notera que l'humidification du gaz servant essentiellement à assurer une humidité suffisante au niveau de l'objet à stériliser, il est également possible, comme l'illustrent les figures 1b et 1c, d'alimenter la zone de production du plasma avec un premier gaz non biocide 12 (contenant au moins 10% d'oxygène et 10% d'azote, avantageusement l'air ambiant) et la zone de stérilisation avec un second gaz non biocide 26 (avantageusement l'air ambiant) mais ensuite humidifié (au travers de l'humidificateur 14), de manière à maintenir l'humidité au niveau requis (mesurée alors en entrée de la zone de stérilisation 10b par un second capteur d'humidité 18b placé en sortie de cet humidificateur). On notera, que le premier capteur 18a peut être maintenu en entrée de la zone de production du plasma 10a, notamment lorsque le premier gaz n'est pas délivré à humidité constante (par exemple s'il s'agit de l'air ambiant). Les premier et second gaz non biocides peuvent être de même nature (figure 1b) ou de nature différente (figure 1c). Bien entendu, un dispositif de contrôle de débit 28 peut aussi être placé en sortie de la seconde source de gaz 26.

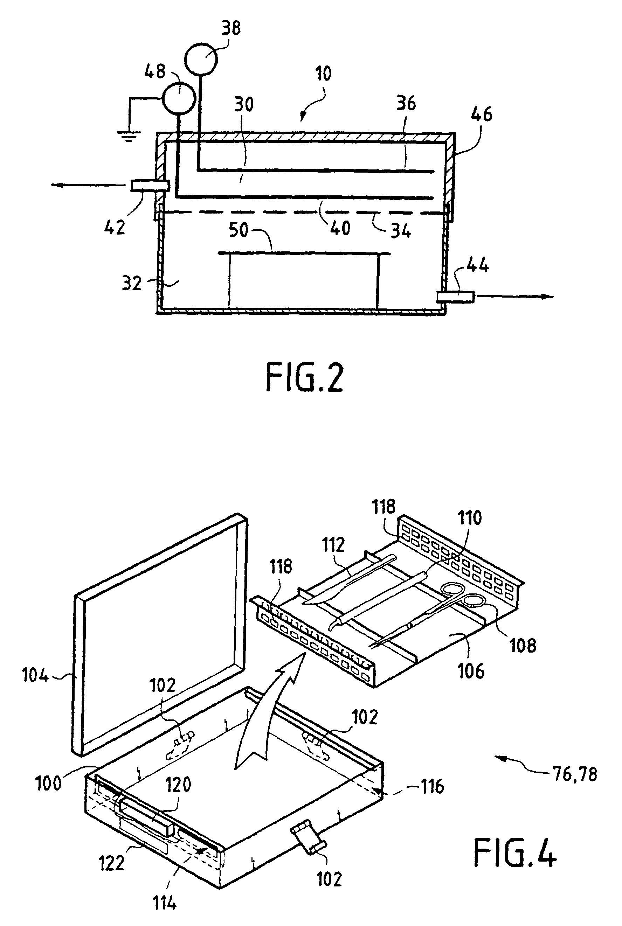

La figure 2 est un schéma de principe plus détaillé de l'enceinte de traitement 10 qui est formée d'une zone de production du plasma 30 et d'une zone de stérilisation 32. Les deux zones sont éventuellement séparées par une grille métallique 34, la zone de stérilisation pouvant présenter une configuration fermée ou bien ouverte dans le cas de traitement de surfaces. L'objet à stériliser 50 est placé sur un support, qui doit permettre la circulation de l'agent stérilisant sur toute sa surface, dans la zone de stérilisation 32, c'est à dire en dehors de la zone de production du plasma (zone inter-électrodes 30 de création de la décharge).

Dans l'exemple illustré, l'alimentation se fait par un mélange gazeux humide et la zone de production du plasma comprend d'une part un orifice d'entrée pour l'arrivé du gaz vecteur et d'autre part deux électrodes, une éiectrode haute tension 36 alimentée par un générateur haute tension basse fréquence 38 et une électrode de masse 40, destinées à produire entre elles une décharge électrique dite décharge « couronne ». La décharge couronne est caractérisée par l'utilisation de deux électrodes ayant un rayon de courbure très différent. L'augmentation du champ électrique près de l'électrode à faible rayon de courbure permet de diminuer la tension nécessaire à l'apparition de la décharge tout en utilisant une alimentation en tension qui peut être basse fréquence. L'une ou l'autre des électrodes - à faible et fort rayon de courbure - peuvent être connectées à la haute tension, l'autre se trouvant connectée à la masse. Bien entendu, cette configuration d'électrodes n'est en aucune façon limitative et ces électrodes peuvent également se présenter sous la forme d'un réseau d'électrodes parallèles dont le nombre et la disposition dépend de la taille et de la nature de l'objet à stériliser.

L'électrode à faible rayon de courbure 36 (reliée à la haute tension sur le schéma) est métallique, éventuellement recouverte d'un diélectrique, et peut se présenter sous la forme de pointes, d'un fil ou d'un fil comportant des pointes. Lorsque le volume de la zone de stérilisation est important (ce qui est fonction de la taille et du nombre d'objets à stériliser), les électrodes sont avantageusement montées en un réseau d'électrodes parallèles, l'alimentation des électrodes haute tension pouvant alors avoir lieu de façon simultanée ou successive pendant le temps de stérilisation. Le nombre d'électrodes et leur longueur sont déterminés de telle sorte que la production d'espèces chimiques stérilisantes soit suffisante pour obtenir la concentration nécessaire à la stérilisation.

Ces espèces chimiques stérilisantes produites par la décharge couronne dans l'espace inter-électrodes peuvent être amenées dans la zone de stérilisation soit naturellement par le vent électrique créé par la décharge entre les deux électrodes (et qui est orienté de l'électrode à faible rayon de courbure vers l'électrode à fort rayon de courbure), soit par la création d'un écoulement forcé dans l'enceinte de traitement.

L'efficacité sporicide, donc le temps de stérilisation en un endroit donné de l'enceinte de traitement (appelée aussi réacteur) dépend de la vitesse de propagation et de la vitesse locale. Pour un écoulement simple à vitesse constante, les deux valeurs sont égales : il existe donc un optimum en vitesse (et en débit) assurant un temps de stérilisation minimum.

Pour stériliser dans des temps raisonnables des enceintes de grande dimension, il est intéressant de prévoir l'écoulement à l'intérieur de l'enceinte de manière à accélérer le gaz durant sa phase de propagation et de le ralentir localement au niveau de la surface à traiter. De cette manière, vitesse de propagation et vitesse locale peuvent être indépendamment optimisées pour diminuer le temps de stérilisation.

L'électrode à fort rayon de courbure 40 (reliée à la masse sur le schéma) est aussi métallique, éventuellement recouverte d'un revêtement diélectrique, ce qui permet d'éviter le passage à l'arc électrique pendant le cycle de stérilisation. Selon les applications, elle peut se présenter, sous la forme d'un plan, sous la forme d'un fil ou sous la forme d'un cylindre plein ou encore sous une forme grillagée pour faciliter l'écoulement des espèces chimiques produites par la décharge. Cette géométrie variable des électrodes permet ainsi différentes applications au procédé : emballage stérilisant, stérilisation de cavités, désinfection des surfaces par exemple. Il convient de noter que les électrodes s'oxydant au cours du temps du fait de la décharge, la zone de production du plasma et/ou les électrodes peuvent être remplacées après un (cas d'un usage unique) ou plusieurs cycles de traitement.

L'orifice d'arrivée du gaz vecteur 42 est situé de préférence près des électrodes 36 et 40 pour optimiser son passage dans l'espace inter-électrodes, la production du plasma étant localisée dans cet espace inter-électrodes. Un orifice de sortie du gaz 44 est situé quant à lui dans la zone de stérilisation 32 en aval de l'objet à traiter par rapport au sens d'écoulement naturel du gaz.

De plus, pour que le système soit compatible avec les normes de compatibilité électromagnétique, la zone de production du plasma à l'intérieur de laquelle se produit la décharge est avantageusement formée par une cage de Faraday 46 pour limiter les interférences créées par cette décharge.

En série avec l'électrode de masse ou avec l'électrode haute tension est monté un dispositif de mesure de courant 48 au niveau duquel il est possible de mesurer un courant de décharge. La mesure de ce courant permet d'ajuster et de contrôler le niveau de tension nécessaire à l'établissement de la décharge dans l'espace inter-électrodes. En mesurant le courant en amont et en aval de la décharge, il est possible de vérifier que le courant injecté par la source est bien celui repartant vers l'électrode de masse (protection différentielle).

Le générateur haute tension 38 est un générateur basse fréquence. Le signal de tension appliqué sur l'électrode 36 peut être continu ou en créneaux, de signe positif ou négatif, alternatif ou même pulsé en temps.

La production de différentes espèces gazeuses chimiques dépend du régime de décharge. Pour obtenir un régime de décharge couronne efficace du point de vue de la stérilisation, le signal de tension doit être d'un signe approprié à la géométrie des électrodes. Le signal de tension est de préférence continu positif avec une électrode haute tension comprenant une ou plusieurs pointes. Il est de préférence continu négatif avec une électrode haute tension de type filaire. Avec des électrodes recouvertes d'un diélectrique, il est choisi de préférence alternatif.

Le niveau de tension nécessaire à l'établissement d'une décharge électrique dans l'espace inter-électrodes dépend du diamètre de l'électrode haute tension et de la distance inter-électrodes. Par exemple, il a été mesuré que si on applique une tension continue positive de 12 kV sur un fil de diamètre 0,125 mm, avec une distance inter-électrodes de 10 mm, on obtient une décharge couronne délivrant un courant de décharge de 50 µA/cm. On notera que cette décharge électrique peut être maintenue pendant une fraction seulement du cycle de stérilisation.

Pour un type de signal haute tension donné défini par sa polarité, sa forme et sa fréquence, le régime de décharge et la production d'espèces chimiques dépendent également de l'intensité du courant.

L'impédance effective de la décharge donnant la relation entre courant et tension peut évoluer au cours du temps. La régulation se fait de préférence à courant constant pour maintenir le même régime de décharge et la même production d'espèces chimiques afin d'assurer un effet stérilisant constant. Pour des électrodes linéaires, le régime de décharge et la production d'espèces chimiques sont une fonction du courant par unité de longueur.

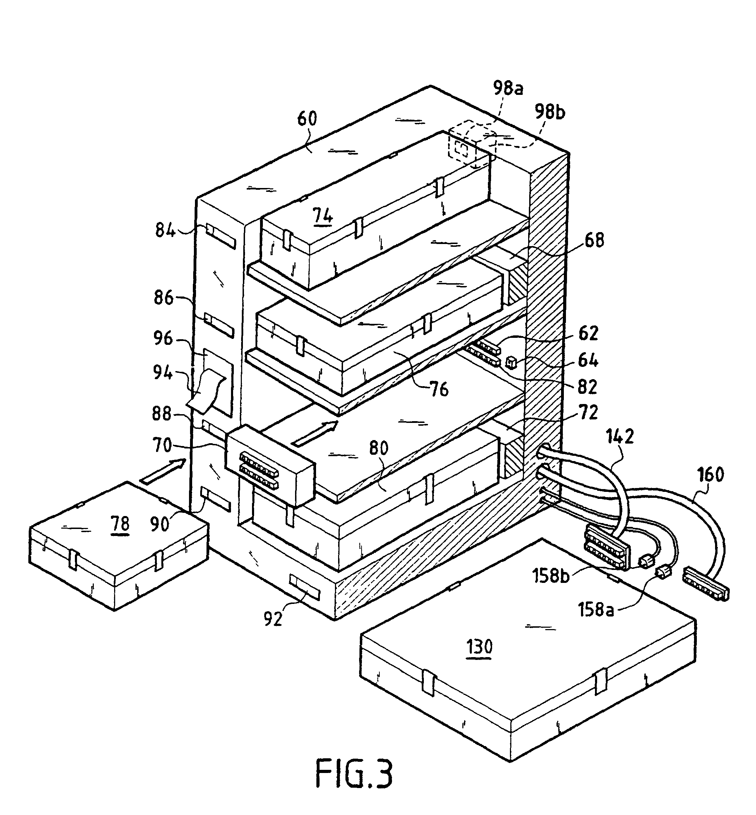

Un premier exemple de réalisation d'un dispositif de stérilisation selon l'invention est illustré à la figure 3 qui montre un ensemble modulaire avec une unité centrale à laquelle sont reliés différents types d'enceintes de traitement. Cet ensemble modulaire permet de proposer un grand nombre de configurations qui se différencient par le type d'enceinte utilisé et la manière dont cette enceinte est reliée à l'unité centrale. Il est ainsi possible de proposer une gamme d'enceintes adaptées aux besoins de chaque utilisateur en fonction du type d'objets à stériliser.

Selon l'invention, cet ensemble modulaire comporte une unité centrale commune contenant la source de mélange gazeux, la chambre d'humidification et l'alimentation haute tension. L'unité centrale 60 comporte une ou plusieurs sorties de gaz (par exemple 62) et un nombre correspondant de sorties haute tension (par exemple 64) alimentant une ou plusieurs enceintes de traitement comportant chacune une zone de production du plasma 68, 70, 72 correspondant à la zone de production du plasma 30 et fournissant du gaz stérilisant à une zone de stérilisation 76, 78, 80 correspondant à la zone de stérilisation 32 contenant les objets devant être traités. Les zones de production du plasma et de stérilisation peuvent former deux zones distinctes d'une même enceinte (par exemple les enceintes 74 et 130), ou elles peuvent intégrer chacune une enceinte séparée appelée alors enceinte de production du plasma (dans le cas des enceintes 68, 70, 72) ou enceinte de stérilisation (pour les enceintes 76, 78, 80). Avantageusement, tout ou partie de l'ensemble du dispositif est placé dans une cage de Faraday pour limiter les interférences créées par la décharge.

Cette configuration modulaire permet de traiter, simultanément ou non, un ensemble d'enceintes adaptées en nombre, forme et volume à ces objets à partir d'une seule unité centrale comportant une ou plusieurs alimentations haute tension et d'un seul système de gestion du gaz (assurant l'alimentation et la récupération) contenu dans l'unité centrale 60. Le gaz après traitement est ensuite retourné à cette unité centrale par une ou plusieurs entrées d'évacuation du gaz (par exemple 82).

Des moyens d'indication et de commande 84, 86, 88, 90 disposés sur l'unité centrale 60 en regard des enceintes correspondantes auxquelles ils sont associés permettent d'assurer un contrôle individuel de chaque enceinte en assurant le démarrage du cycle de stérilisation et le réglage du temps de traitement, en réglant le débit de consigne et le courant de régulation appropriés, et éventuellement en définissant la composition du mélange gazeux à mettre en oeuvre. Le suivi des différentes commandes est assuré par la sortie d'une étiquette imprimée 94 sur une imprimante 96 intégrée à l'unité centrale 60. Pour chaque enceinte, qui porte un numéro d'identification spécifique, il est ainsi possible de mentionner sur cette étiquette la date du traitement et les paramètres du cycle de stérilisation, notamment sa durée. Les enceintes peuvent être munies d'un système d'identification automatique, par exemple à base de codes barres ou d'étiquettes électroniques 98a de type RFID (RadioFrequency IDdentification) ou de type IRC (InfraRed Communication). qui permet par l'intermédiaire d'un lecteur correspondant 98b de l'unité centrale de déterminer automatiquement les valeurs de débit de consigne et le courant de régulation appropriés et de calculer le temps de stérilisation. Ces étiquettes électroniques peuvent être placées à l'intérieur de l'enceinte et être avantageusement munies de capteurs permettant de contrôler le cycle de stérilisation, notamment des capteurs de mesure chimiques pour par exemple la mesure de l'humidité, de l'ozone, du PH, ou du taux en dioxyde d'azote.

La zone de stérilisation peut être de taille variable ou standardisée selon les besoins de l'utilisateur. En adaptant la forme et le volume de la zone aux objets à stériliser, il est possible d'optimiser la circulation de l'agent stérilisant (sa vitesse d'écoulement et sa concentration) autour des objets et ainsi d'assurer un traitement homogène.

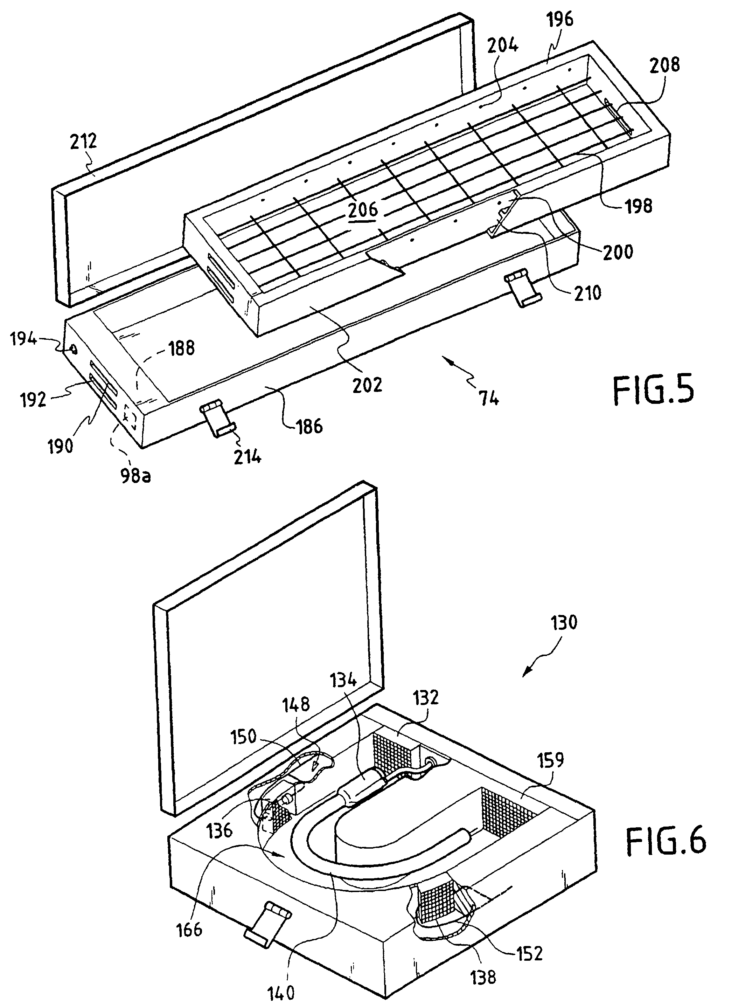

Les figures 4 à 7 illustrent différents exemples de configuration d'enceintes.

Sur la figure 4, l'enceinte de stérilisation 76, 78 est réalisée indépendamment de l'enceinte de production du plasma 68, 70. Elle comporte, dans un boîtier 100 dont une fermeture hermétique peut être assurée par exemple par des clips de sécurité 102 venant en prise sur un couvercle 104 de ce boîtier, une plaque support 106 pour les objets à stériliser, par exemple une paire de ciseaux 108, un scalpel 110 et un couteau 112. Cette plaque support, qui pourra être réalisée dans un matériau poreux pour faciliter la circulation des espèces chimiques stérilisantes, sépare le boîtier en deux espaces superposés, un espace supérieur 114 et un espace inférieur 116, dans lesquels circulera successivement ces espèces stérilisantes. Cette circulation est rendue possible du fait d'orifices présents sur cette plaque ou sur le boîtier et dont la configuration est déterminée pour assurer un écoulement laminaire dans la zone de stérilisation. Par exemple, des orifices 118 de passage des espèces chimiques peuvent être prévus au niveau de cette plaque support ainsi que des orifices d'entrée 120 et de sortie 122 des espèces chimiques stérilisantes au niveau du boîtier, respectivement dans l'espace supérieur 114 et l'espace inférieur 116. Ces orifices d'entrée et de sortie seront munis de clapets anti-retour et/ou pourvus avantageusement de filtres anti-bactériens pour assurer l'étanchéité de l'espace intérieur du boîtier après traitement. L'étanchéité peut également être assurée par un simple système de fermeture manuel utilisant par exemple le clampage d'un tuyau. La relation entre débit et vitesse étant une fonction du taux de remplissage de l'enceinte, il est possible de mesurer la vitesse d'écoulement dans l'enceinte après remplissage à l'aide d'un capteur de vitesse placé dans l'enceinte afin d'ajuster le débit à la valeur permettant d'obtenir la vitesse d'écoulement souhaitée. Ce capteur peut avantageusement constituer un des éléments de l'étiquette électronique 98a.

Pour conserver le caractère stérile à un objet jusqu'à son utilisation, on notera qu'il est en principe nécessaire d'emballer l'objet avant le traitement. Les espèces chimiques doivent alors diffuser à l'intérieur de l'emballage et ne pas interagir avec celui-ci. Dans le cas de la stérilisation par la chaleur, la vapeur d'eau diffuse à travers l'emballage sans interagir avec celui-ci mais, dans le cas de stérilisation par les gaz, il faut en principe développer un emballage de composition spécifique qui n'interagit pas avec les espèces chimiques du plasma.

La simplicité de l'ensemble modulaire décrit précédemment permet d'éviter ce problème car le boîtier forme naturellement un emballage stérilisant, de plus facilement transportable et réutilisable. Bien entendu, il doit être maintenu fermé après le cycle de stérilisation pour cette fonction de protection bactériologique (emballage). Mais, s'il doit être ouvert pour utiliser tout ou une partie des objets qu'il renferme, il est alors nécessaire d'engager un nouveau cycle de stérilisation en replaçant le boîtier dans l'unité centrale. On notera en outre que ce boîtier assure aussi une fonction de protection mécanique (mallette de transport ou de stockage).

Revenons à la figure 3 qui montre également deux autres configurations possibles de l'enceinte de traitement pour effectuer la stérilisation d'objets. Dans chacune de ces configurations, la zone de production du plasma (génération de la décharge) et la zone de stérilisation sont placées dans une même enceinte 74, 130 alimentée directement en gaz vecteur et en haute tension à partir de l'unité centrale 60. Bien entendu. ces enceintes peuvent contenir une ou plusieurs zones de production du plasma.

La figure 5 illustre un premier exemple de réalisation d'une telle enceinte pouvant étre utilisée pour un usage général. L'enceinte 74 qui forme emballage après traitement comporte un boîtier 186 de forme standardisée, existant éventuellement en différentes tailles. Elle comprend une ou plusieurs zones de production du plasma 188 intégrées directement dans ce boîtier, des connecteurs pour les alimentations en gaz 190, 192 et en tension 194, ainsi qu'une étiquette électronique 98a. L'étanchéité des connexions gaz avec l'extérieur peut être assurée comme dans le mode de réalisation précédent, mais un simple filtre bactériologique laissant passer l'air et l'humidité peut être suffisant. Ce filtre n'a pas besoin d'être compatible avec les espèces chimiques stérilisantes puisque la décharge est intégrée à l'emballage.

A l'intérieur du boîtier 186 est logé un plateau support 196 choisi par l'opérateur en fonction du ou des d'objets à stériliser. Ce support permet de transformer une enceinte de forme standard en une enceinte adaptée spécifiquement à un ou des objets particuliers. En effet, les connecteurs et le système d'étanchéité étant entièrement contenus dans le boîtier, le support interne 196 peut être de réalisation très simple, directement adapté au volume et à la forme de l'objet à traiter, avec une géométrie définie de manière à optimiser l'écoulement en assurant une vitesse de propagation maximum pour une vitesse locale minimum près des objets à stériliser. Il comprend une grille 198 sur laquelle sont posés le ou les objets et des zones de propagation de faible section 200 situées dans une double paroi 202 de ce plateau support permettant d'accélérer le gaz jusqu'à des alimentations locales en gaz 204 débouchant dans la zone de stérilisation 206 au niveau de différentes parties du ou des objets à traiter de façon à assurer une stérilisation sur toutes ses faces. Les effluents sont récupérés en bout d'enceinte par un passage 208 et renvoyées via des zones de retour 210 contenues dans la double paroi et via l'orifice de récupération 192 vers la zone d'alimentation principale de l'enceinte. La propagation des espèces chimiques vers les alimentations locales peut également être assurée par de simples tubes de diamètre adapté. L'enceinte est bien entendu recouverte par un couvercle 212 maintenu hermétiquement fermé par des clips 214.

Il est à noter que ce plateau support 196 peut également être utilisé dans l'enceinte 100 et qu'inversement la plaque support 106 de cette dernière enceinte peut être utilisée dans l'enceinte 74.

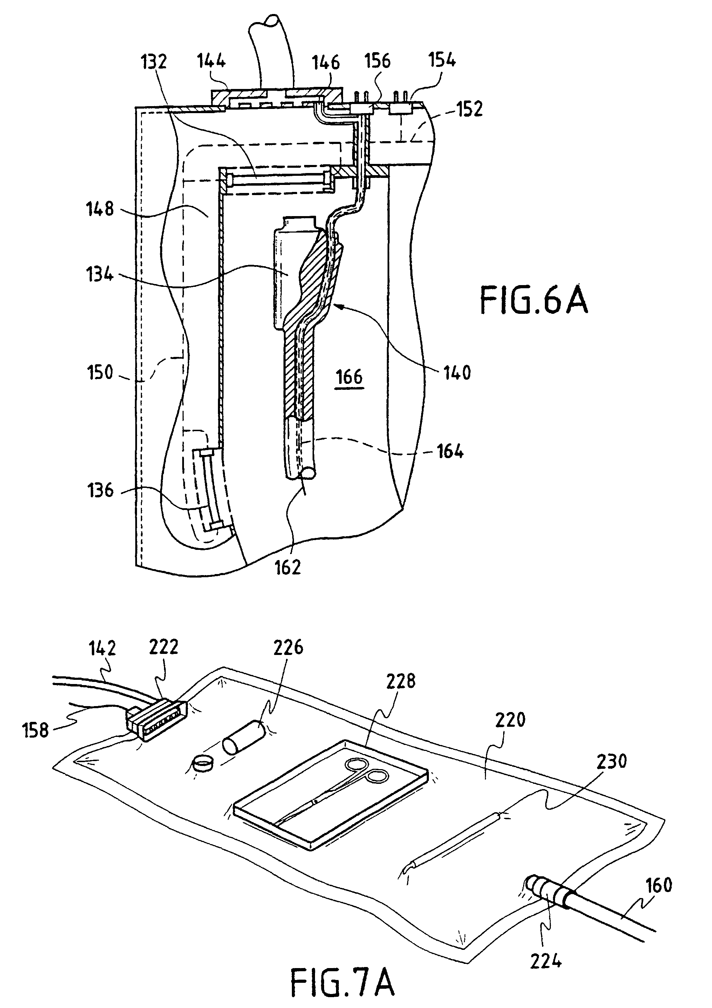

Les figures 6 et 6a illustrent un second exemple de réalisation d'une enceinte de traitement plus spécialement adaptée à la stérilisation d'un endoscope et munie de trois zones indépendantes de production de plasma.

Cette enceinte de traitement 130 se caractérise par une géométrie particulière des électrodes constituant la zone de production du plasma qui permet également la production in-situ des espèces chimiques stérilisantes. En effet, avec les techniques de stérilisation conventionnelles, les zones internes des objets peuvent poser des difficultés de stérilisation si les principes actifs ont du mal à les atteindre. Le problème est particulièrement vrai pour les cavités ou l'intérieur des tubes, comme par exemple dans le cas des canaux des endoscopes. Or, le procédé de stérilisation de l'invention qui peut parfaitement être appliqué à la stérilisation de ces cavités permet également de résoudre simplement ce problème d'accès à ces zones internes.

L'enceinte 130 se présente sous la forme d'un boîtier pouvant être hermétiquement fermé et dont l'espace intérieur (la zone de stérilisation proprement dite) est arrangé en fonction de la forme de l'objet à traiter. Ainsi, dans l'exemple illustré, l'endoscope étant replié à plat dans l'enceinte, il est défini trois zones distinctes de production du plasma, l'une 132 au niveau de sa tête 134 et deux autres 136, 138 réparties régulièrement le long de la surface externe de son canal 140. Le gaz vecteur est amené au boîtier de l'unité centrale 60 par au moins une liaison externe 142 et est redistribué aux zones de production du plasma respectivement par des canalisations internes 144, 146, 148. Les électrodes de ces zones de production du plasma sont reliées notamment par des liaisons 150, 152 à un premier connecteur haute tension externe 154 en liaison avec un premier connecteur compatible correspondant 158a de l'unité centrale commune 60. Une liaison 160 permet l'évacuation vers cette unité centrale 60 du gaz après traitement. Pour la stérilisation de la surface interne du canal de l'endoscope, il est en outre prévu un système d'électrodes filaires spécifiques (voir la figure 6a). L'électrode haute tension 162, alimentée à partir d'un second connecteur 156 relié également à un connecteur 158b de l'unité centrale, peut se présenter sous la forme d'un fil comportant éventuellement des pointes et disposé le long de l'axe longitudinal de ce canal et l'électrode de masse 164 est avantageusement constituée par un cylindre grillagé placé autour de cette électrode haute tension. Une configuration avec deux électrodes filaires organisées en faisceau est aussi envisageable. Dans tous les cas, pour des raisons de stabilité de la décharge, ces électrodes filaires doivent être revêtues d'un isolant, comme c'est le cas pour un fil émaillé, par exemple. Ce système d'électrodes est placé dans le canal de l'endoscope avant le début du cycle de stérilisation. Toutefois, dans certains cas, il peut être préférable d'intégrer les électrodes de décharge (qui seront éventuellement remplaçable) directement à l'objet à traiter. En effet, il est tout à fait envisageable que la zone de production du plasma soit intégrée à l'objet à traiter dès sa conception et dont elle forme alors une partie. L'évacuation des effluents gazeux de la zone de stérilisation 166 peut être effectuée depuis le connecteur 159 également au travers de la liaison 160. Comme dans l'exemple précédent, des clapets anti-retour et/ou des filtres anti-bactériens sont prévus aux interfaces du boîtier 130 pour assurer son étanchéité après sa déconnexion de l'unité centrale commune 60. Un contrôle individuel de ce boîtier est assuré par un moyen d'indication et de commande 92 placé sur l'unité centrale.

Les modes de réalisation de l'enceinte proposée précédemment utilisent l'enceinte de stérilisation comme emballage du ou des objets à traiter. L'utilisation d'un objet imposant l'ouverture complète de l'enceinte, tous les objets présents même ceux non utilisés devront être de nouveau stérilisés avant une prochaine utilisation.

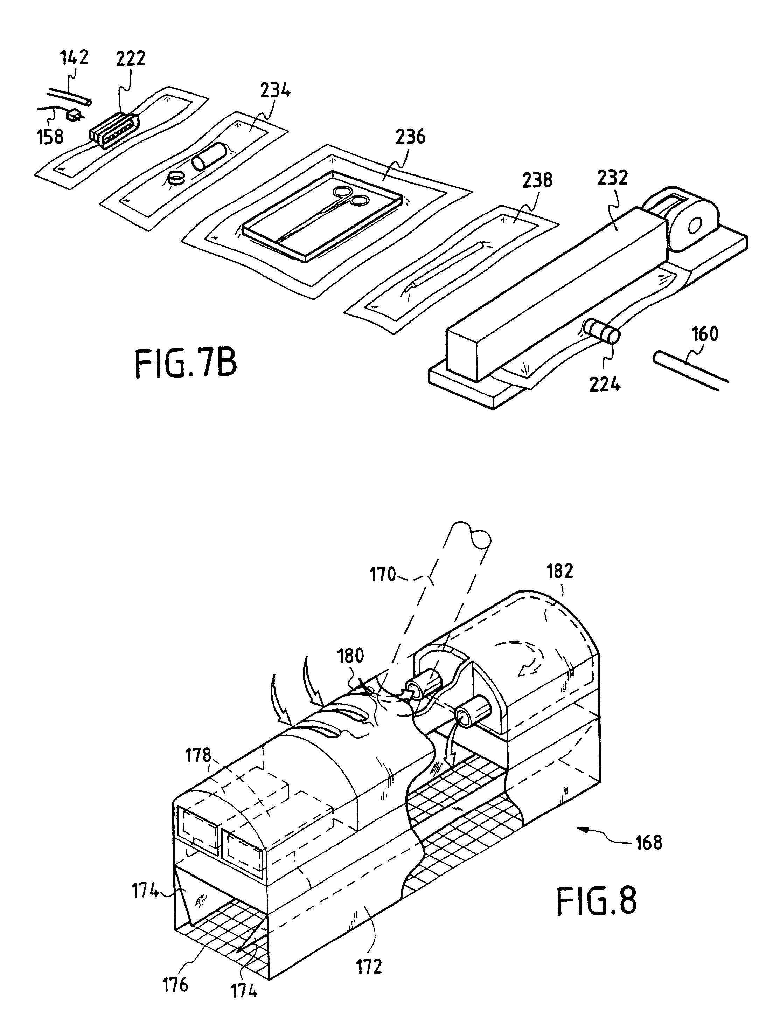

Les figures 7a et 7b proposent encore une autre configuration de l'enceinte de traitement répondant au problème posé par les objets devant être stérilisés simultanément mais utilisés séparément. Dans ce troisième exemple de réalisation, tout ou partie de l'enceinte de traitement est réalisée dans un matériau souple permettant un scellement individuel après le traitement. Ce scellement permet de séparer les objets contenus dans l'enceinte après stérilisation sans risque de contamination et d'utiliser de fait séparément les différents objets.

Dans un mode de réalisation préférentiel, l'enceinte est un sac 220 comportant au moins une face transparente et comportant une ouverture pour pouvoir introduire les différents objets. Le matériau du sac est choisi de manière à permettre des opérations de découpe et de scellement concomitant, par exemple par thermosoudage ou tout autre procédé équivalent. La taille et la forme du sac sont déterminées par l'opérateur en fonction de ses besoins. Ce sac contient une source plasma 222 à une extrémité et un embout d'évacuation 224 à l'autre. La source plasma et l'embout d'évacuation peuvent être scellés sur le sac par l'opérateur ou être intégrés directement au sac lors de sa conception. Après dépôt des objets 226, 228, 230 à traiter, le sac est scellé à ses extrémités de manière à assurer l'étanchéité vers l'extérieur tout en permettant la circulation du gaz d'un bout à l'autre de l'enceinte. Cette circulation peut être facilitée soit en mettant le sac en légère suppression afin de le gonfler soit en utilisant la forme de la source plasma et de l'embout de connexion pour l'écarter. La source plasma et l'embout d'évacuation sont reliés à l'unité centrale 60 respectivement par les liaisons 142, 160. Une liaison électrique 158 assure l'alimentation en haute tension de la source plasma.

Après le cycle de stérilisation, une machine de scellement 232 permet d'isoler les objets les uns des autres dans des sachets stériles individuels 234, 236, 238 et de la source et de l'embout d'évacuation (voir la figure 7b). Cette opération se fait sans risque de contamination de l'extérieur. L'isolation obtenue entre les objets permet une utilisation séparée de ces différents objets. La source plasma et l'embout peuvent être jetés ou recyclés après le traitement. Ce principe peut également être utilisé pour les enceintes rigides en plaçant à l'intérieur d'un sac différentes enceintes, le sac faisant office de couvercle en fin de stérilisation.

Notons enfin, que dans une configuration de base non représentée, tous les éléments nécessaires à la stérilisation (source de gaz vecteur, humidificateur, électrodes, alimentation haute tension) ainsi que l'enceinte de traitement, qui peut contenir un ou plusieurs objets à traiter simultanément, peuvent bien entendu être placés dans un module ou enceinte unique fermé.

Un second exemple de réalisation d'un dispositif de stérilisation par plasma est illustré à la figure 8. Il concerne plus particulièrement la désinfection de surfaces. En effet, le procédé de l'invention permet la conception d'un dispositif de désinfection des surfaces, à l'air ambiant, pouvant être utilisé par exemple pour la désinfection des sols. Les temps nécessaires à la désinfection sont beaucoup plus courts que ceux exigés pour une stérilisation et permettent donc la mise au point d'un système de traitement de surfaces par déplacement de la zone de production du plasma. Dans ce cas, le traitement assure une désinfection poussée vis à vis de tous micro-organismes (bactéries, levures, moisissures et acariens) et il peut être inclus dans un dispositif de nettoyage car les objets à stériliser ne nécessitent pas d'être séchés avant le traitement de stérilisation.

Dans un tel dispositif de désinfection de surfaces, la zone de stérilisation n'est pas constituée par une enceinte fermée mais par la zone libre située immédiatement en aval de la zone de production du plasma. En effet, ce dispositif se présente sous la forme d'un balai 168 avec un manche 170 fixé sur un corps de balai 172 formant une enceinte protectrice pour les différents éléments nécessaires à la génération d'un plasma. Notamment, le système d'électrodes avec son électrode haute tension 174 et son électrode de masse pouvant être constituée d'une simple grille métallique 176. Les électrodes ont les mêmes caractéristiques que celles décrites dans le premier exemple de réalisation, l'électrode haute tension pouvant par exemple être réalisée sous la forme de pointes, de fil ou de fil comportant des pointes. Toutefois, pour des raisons d'efficacité, l'électrode haute tension 174 est ici doublée et forme ainsi un bilame comportant éventuellement des pointes au milieu duquel se fait l'arrivée du mélange gazeux. Par cette structure spécifique, le système d'électrodes crée ainsi deux rideaux de plasma dans l'espace inter-électrodes. Bien entendu, ce système d'électrodes peut être reproduit plusieurs fois en parallèle dans des dispositifs plus grands. L'alimentation des électrodes est effectuée avantageusement à partir d'une ou plusieurs batteries 178 intégrées au corps du balai 172.

Dans ce mode de réalisation, le mélange gazeux est constitué simplement par de l'air ambiant introduit dans le corps de balai par une entrée d'air externe 180 puis humidifié dans une recharge d'eau 182. Un compresseur d'air muni éventuellement d'un dispositif de filtration peut éventuellement être placée juste derrière l'entrée d'air avant la recharge d'eau.

Le principe de fonctionnement de ce balai 168 est analogue à celui régissant les enceintes précédentes. Le mélange gazeux constitué par l'air humidifié est transformé en plasma entre les électrodes et les espèces chimiques produites par la décharge couronne dans l'espace inter-électrodes sont véhiculées d'une part par le vent électrique qui est orienté de l'électrode à faible rayon de courbure (bilame de l'électrode à haute tension) vers l'électrode à fort rayon de courbure (grille de l'électrode de masse) vers la surface à désinfecter située en aval de l'électrode de masse et d'autre part par une circulation forcée. Il se produit alors une désinfection de la surface, l'évacuation du gaz se faisant naturellement au travers de la grille 176. Ce dispositif de désinfection peut comporter un système de roulement (non représenté) pour parcourir toute la surface considérée.

On notera que la circulation du gaz vecteur doit être répartie uniformément dans le système d'électrodes et notamment entre les deux électrodes à haute tension pour favoriser le renouvellement d'air et forcer le passage du gaz vecteur à travers les deux rideaux de plasma.

L'exemple suivant permettra de mieux apprécier l'efficacité du procédé de stérilisation par plasma selon l'invention dont l'effet stérilisant est comparé selon la composition du gaz vecteur et selon le type de spores bactériennes.

Des essais ont été effectués avec des indicateurs biologiques contenant des micro-organismes particulièrement résistants à la stérilisation par les gaz. Il s'agit de spores de *Bacillus subtilis* (provenant de la Collection de l'institut Pasteur, CIP 77.18) et de spores de *Bacillus stearothermophilus* (provenant de la Collection de l'Institut Pasteur, CIP 52.81) inoculées sur des lamelles en verre. Chaque échantillon contient 10⁶ spores du bacille.

Chaque indicateur biologique est placé dans la zone de stérilisation et exposé à un cycle de traitement. Pendant le traitement, le débit du mélange gazeux est de 0,2 litre / mn dans une zone de traitement de 0,5 litre à la pression atmosphérique et à la température ambiante. L'électrode à haute tension est une lame en acier de 5 cm de longueur portant des pointes sur toute sa longueur. L'électrode de masse est un disque en acier de 5 cm de diamètre. La distance inter-électrodes est de 10 mm. La décharge couronne est obtenue en appliquant une tension continue positive de 11 kV à l'électrode à haute tension.

Après avoir exposé les indicateurs biologiques à un cycle de traitement de 20 minutes, ils sont transférés stérilement sur un milieu nutritif. Les indicateurs biologiques contenant des spores de *Bacillus subtilis* sont incubés à 37°C pendant 15 heures. Les indicateurs biologiques contenant des spores de *Bacillus stearothermophilus* sont incubés à 56°C pendant 30 heures.

| Composition du gaz vecteur | Humidité | % de spores de *Bacillus subtilis* restantes après 20 minutes de traitement | % de spores de *Bacillus stearothermophilus* restantes après 20 minutes de traitement |
|---|---|---|---|
| 20 % de O₂ dans l'azote | 100 % | * | 0.01 |
| | 0 % | 100 | 100 |
| 20 % de O₂ dans l'argon | 100 % | 0.05 | 50 |
| | 0 % | 100 | 100 |

| | | | |
|---|---|---|---|
| * moins de 1 spore restante | | | |

On observe clairement que le procédé selon l'invention est particulièrement efficace lorsque le gaz vecteur contient une forte humidité relative. Au contraire, avec un procédé classique utilisant un gaz rare, tel que l'argon, l'effet sur les spores de *Bacillus subtilis* est relativement faible et voire très faible sur les spores de *Bacillus stearothermophilus*, ce qui ne permet d'obtenir un niveau de qualité suffisant pour une stérilisation.

Ainsi, l'invention concerne un procédé de stérilisation par plasma en post-décharge à température ambiante et à pression atmosphérique (ou une pression proche de la pression atmosphérique), au moyen d'une décharge couronne établie dans un mélange gazeux non-biocide contenant de l'oxygène et de l'azote, et en présence d'humidité. Ce procédé a un effet sporicide sur des spores de *Bacillus subtilis* comme de *Bacillus stearothermophilus* et cela en moins d'une heure. Ce procédé permet en outre de stériliser des objets et des surfaces quelles que soient leur forme et leur nature, métallique, composite et thermosensible.

La stérilisation par plasma de décharge couronne apparaît comme une technique innovante utilisable à la température ambiante, dans un gaz à la pression atmosphérique et sans résidus toxiques. Le procédé ainsi décrit est à la fois simple de conception, car l'enceinte n'a pas besoin de résister à des différences de pression importante, le système d'alimentation haute tension est à basse fréquence et le système d'alimentation en gaz est simplifié, et simple d'utilisation, car il n'y a pas de produits chimiques à manipuler avant et après le cycle de stérilisation et les risques de pollution sont limités. Il présente en outre un faible coût de fabrication du fait du recours à des atmosphères gazeuses et des structures d'électrodes simples.

Les applications proposées concernent essentiellement le domaine médical mais le procédé peut être étendu à bien d'autres applications industrielles, par exemple dans les domaines agroalimentaire ou pharmaceutique. La grande souplesse du dispositif permet en outre la stérilisation de surfaces des emballages, des produits ou des matériels de production, à la désinfection des zones de confinement ou de transfert. Le dispositif peut aussi être utilisé pour la décontamination des surfaces internes des systèmes d'air conditionné.

## Revendications

1. Procédé de stérilisation par plasma à pression égale ou sensiblement égale à la pression atmosphérique d'au moins un objet dans lequel :
a) on place le ou les objets à traiter (50) dans une enceinte de traitement (10) soumise sensiblement à la pression atmosphérique,
b) on introduit dans cette enceinte de traitement un ou plusieurs mélanges gazeux non-biocides dont l'un au moins contient de l'humidité,
c) on crée un plasma produisant des espèces chimiques à partir d'un des mélanges gazeux en générant au moyen d'une alimentation haute tension (38) une décharge électrique entre une électrode à haute tension (36) et une électrode de masse (40), ces deux électrodes étant placées dans cette enceinte de traitement,
d) on achemine les espèces chimiques du plasma en dehors de la zone inter-électrodes (30), vers la surface du ou des objets à traiter (50), et
e) on évacue les résidus gazeux résultant du traitement hors de l'enceinte de traitement.

2. Procédé selon la revendication 1, **caractérisé en ce** l'humidité est introduite directement au niveau du ou des objets à traiter (50).

3. Procédé selon la revendication 1, **caractérisé en ce que** l'humidité est introduite au niveau de la zone inter-électrodes (30).

4. Procédé selon l'une quelconque des revendications 1 à 3, **caractérisé en ce que** le mélange gazeux contient au moins 10% d'oxygène et 10% d'azote.

5. Procédé selon la revendication 4, **caractérisé en ce que** le mélange gazeux est constitué par l'air ambiant.

6. Procédé selon l'une quelconque des revendications 1 à 5, **caractérisé en ce que** le taux d'humidité relative au niveau du ou des objets à traiter (50) est compris entre 50% et 100%.

7. Procédé selon la revendication 6, **caractérisé en ce que** le taux d'humidité relative au niveau du ou des objets à traiter (50) est supérieur ou égal à 90%.

8. Procédé selon la revendication 1, **caractérisé en ce que** l'étape b) d'introduction du ou des mélanges gazeux dans l'enceinte de traitement (10) est effectuée de façon continue ou par intermittence.

9. Procédé selon la revendication 8, **caractérisé en ce qu'**il est réalisé un contrôle du débit du ou des mélanges gazeux entrant dans l'enceinte de traitement (10).

10. Procédé selon la revendication 1, **caractérisé en ce que** l'étape c) de création du plasma est précédée par une étape de circulation forcée du ou des mélanges gazeux dans l'enceinte de traitement (10).

11. Procédé selon la revendication 1, **caractérisé en ce que** l'étape d) d'acheminement des espèces chimiques du plasma vers la surface à traiter (50) est obtenue par l'utilisation du vent électrique créé par la décharge entre les deux électrodes (36, 40).

12. Procédé selon la revendication 1, **caractérisé en ce que** l'étape d) d'acheminement des espèces chimiques du plasma vers la surface à traiter (50) est obtenue par la création d'un écoulement forcé dans l'enceinte de traitement (10).

13. Dispositif de stérilisation par plasma d'au moins un objet, **caractérisé en ce qu'**il comporte :
- une première source de gaz (12) contenant un mélange gazeux non-biocide,
- au moins une enceinte de traitement (10) soumise à la pression atmosphérique comportant au moins une zone de stérilisation (10b, 32) dans laquelle sont placés le ou les objets à traiter (50), cette enceinte comportant en outre, dans au moins une zone de production du plasma (10a, 30) distincte de la zone de stérilisation, au moins deux électrodes (36, 40) reliées à une alimentation haute tension (38) pour créer un plasma produisant des espèces chimiques en générant une décharge électrique entre ces électrodes à partir du mélange gazeux introduit dans la zone de production du plasma, les espèces chimiques du plasma étant acheminées en dehors de la zone de production du plasma vers la surface de
ou des objets à traiter et les résidus gazeux résultant du traitement étant évacués vers un système de récupération (22) par un orifice de sortie (44) de cette enceinte, et
- une chambre d'humidification (14) reliée en sortie d'une seconde source de gaz (12, 26) pour maintenir un taux d'humidité déterminé au niveau du ou des objets à traiter (50).

14. Dispositif selon la revendication 13, **caractérisé en ce que** les premiére et seconde sources de gaz forment une source unique de gaz (12).

15. Dispositif selon la revendication 14, **caractérisé en ce que** la zone de production du plasma (10a, 30) est reliée à cette source unique de gaz au travers de la chambre d'humidification (14).

16. Dispositif selon la revendication 14, **caractérisé en ce que** la zone de production du plasma (10a, 30) est reliée directement à cette source unique de gaz (12), la zone de stérilisation (10b, 32) étant reliée à cette source unique de gaz au travers de la chambre d'humidification (14).

17. Dispositif selon la revendication 13, **caractérisé en ce que** la zone de stérilisation (10b, 32) est reliée à la seconde source de gaz (26) au travers de la chambre d'humidification (14), la zone de production du plasma (10a, 30) étant reliée directement à la première source de gaz (12).

18. Dispositif selon la revendication 16 ou la revendication 17, **caractérisé en ce qu'**il comporte un second capteur d'humidité relative (18b) placé en entrée de la zone de stérilisation (10b, 32).

19. Dispositif selon l'une quelconque des revendications 15 à 17, **caractérisé en ce qu'**il comporte un premier capteur d'humidité relative (18a) placé en entrée de la zone de production du plasma (10a, 30).

20. Dispositif selon l'une quelconque des revendications 13 à 19, **caractérisé en ce que** le mélange gazeux contient au moins 10% d'oxygène et 10% d'azote.

21. Dispositif selon la revendication 20, **caractérisé en ce que** le mélange gazeux est constitué par de l'air ambiant.

22. Dispositif selon la revendication 21, **caractérisé en ce que** l'air ambiant est comprimé préalablement à son humidification.

23. Dispositif selon l'une quelconque des revendications 13 à 22, **caractérisé en ce que** la zone de stérilisation (10b, 32) comprend un taux d'humidité relative compris entre 50% et 100%, avantageusement supérieur ou égal à 90%.

24. Dispositif selon l'une quelconque des revendications 13 à 23, **caractérisé en ce qu'**il comporte au moins une électrode à fort rayon de courbure et une électrode à faible rayon de courbure, l'une étant une électrode à haute tension (36) et l'autre étant une électrode de masse (40).

25. Dispositif selon la revendication 24, **caractérisé en ce que** l'électrode à faible rayon de courbure est une électrode métallique pouvant présenter l'une des formes suivantes : fil, pointes ou fil comportant des pointes.

26. Dispositif selon la revendication 24, **caractérisé en ce que** l'électrode à fort rayon de courbure est une électrode métallique pouvant présenter l'une des formes suivantes : fil, plan, cylindre plein ou grille.

27. Dispositif selon la revendication 25 ou la revendication 26, **caractérisé en ce que** l'une ou l'autre des deux électrodes, ou les deux, sont recouvertes d'un revêtement diélectrique.

28. Dispositif selon la revendication 25 et la revendication 26, **caractérisé en ce que** l'électrode à haute tension est constituée par un fil (162) et **en ce que** l'électrode de masse est constituée par un cylindre grillagé (164) entourant ce fil.

29. Dispositif selon la revendication 24, **caractérisé en ce que** les électrodes sont montées en un réseau d'électrodes parallèles, l'alimentation des électrodes à haute tension étant effectuée successivement ou de façon simultanée.

30. Dispositif selon l'une quelconque des revendications 13 à 29, **caractérisé en ce que** les électrodes sont à usage limité.

31. Dispositif selon l'une quelconque des revendications 13 à 30, **caractérisé en ce que** l'alimentation haute tension (38) est assurée par un générateur basse fréquence délivrant une tension continue, en créneaux, alternative ou pulsée en temps.

32. Dispositif selon la revendication 13, **caractérisé en ce qu'**il comporte plusieurs enceintes de traitement, chaque enceinte de traitement comportant au moins une zone de production de plasma (68 ; 70 ; 72 ; 132, 136, 138 ; 188) reliée de façon fixe ou non à au moins une zone de stérilisation (76 ; 78 ; 80 ; 166 ; 206), les zones de production de plasma étant reliées à une unité centrale commune (60) contenant au moins la première source de gaz non-biocide (12), la chambre d'humidification (14), le système récupérateur de résidus gazeux (22) et l'alimentation haute tension (38).

33. Dispositif selon la revendication 32, **caractérisé en ce que** la zone de stérilisation (166 ; 206) de l'enceinte de traitement (74, 130) présente une forme spécialement adaptée à ou aux objets à stériliser (134, 140 ; 226, 228, 230) afin de limiter la production d'espèces chimiques nécessaires à la stérilisation et d'optimiser la vitesse d'écoulement et la concentration de ces espèces stérilisantes autour de l'objet.

34. Dispositif selon la revendication 33. **caractérisé en ce que** l'enceinte de traitement (74) comporte un boîtier (186) de forme standard et contenant la zone de production du plasma (188), la zone de stérilisation étant formée par un support amovible (196) spécialement adapté à ou aux objets à traiter et logé dans ce boîtier.

35. Dispositif selon la revendication 32 ou la revendication 34, **caractérisé en ce que** la zone de stérilisation comprend des zones de propagation (200) de faible section permettant d'accélérer les espèces chimiques provenant de la zone de production du plasma (188) vers différentes parties du ou des objets à traiter.

36. Dispositif selon la revendication 32, **caractérisé en ce que** la zone de production du plasma est intégrée à l'objet à traiter dont elle en forme une partie.

37. Dispositif selon la revendication 32, **caractérisé en ce que** la zone de stérilisation est séparée de la zone de production du plasma et forme une enceinte indépendante (76, 78, 80).

38. Dispositif selon la revendication 32 ou la revendication 37, **caractérisé en ce que** une ou plusieurs des enceintes de traitement ou de stérilisation constituent un emballage autonome réutilisable, en forme de mallette de transport, permettant de conserver l'état stérile après traitement.

39. Dispositif selon la revendication 32 ou la revendication 37, **caractérisé en ce que** une ou plusieurs des enceintes de traitement ou de stérilisation constituent un emballage perdu, en forme de sac souple (220), la zone de stérilisation pouvant être séparée en plusieurs zones individualisées après le traitement (234, 236, 238), par découpe et scellement concomitant de parties déterminées de ce sac.

40. Dispositif selon l'une quelconque des revendications 32 à 39, **caractérisé en ce que** tout ou partie du dispositif est placé dans une cage de Faraday.

41. Dispositif selon la revendication 32, **caractérisé en ce que** l'unité centrale commune (60) comporte des moyens d'indication et de commande (84, 86, 88, 90) associés à chaque enceinte de stérilisation pour assurer un contrôle individuel de la stérilisation des objets qu'elle contient.

42. Dispositif selon la revendication 41, **caractérisé en ce que** l'unité centrale commune (60) comporte des moyens d'impression (96) pour imprimer une étiquette (94) sur lequel seront imprimés, pour chaque enceinte de stérilisation reliée à cette unité centrale, un numéro d'identification spécifique à chaque enceinte ainsi que la date du traitement et les paramètres du cycle de stérilisation effectué.

43. Dispositif selon la revendication 32, **caractérisé en ce que** l'enceinte de traitement ou de stérilisation est munie d'une étiquette électronique (98a) qui permet par l'intermédiaire d'un lecteur correspondant (98b) de l'unité centrale (60) de déterminer automatiquement les valeurs de débit de consigne et le courant de régulation adaptés à ou aux objets à traiter et de calculer le temps nécessaire à la stérilisation de ces objets.

44. Dispositif selon la revendication 43, **caractérisé en ce que** l'étiquette électronique comporte un capteur de vitesse pour mesurer la vitesse d'écoulement des espèces chimiques du plasma dans l'enceinte.

45. Dispositif selon la revendication 43, **caractérisé en ce que** l'étiquette électronique comporte un capteur de mesure chimique.

46. Application du dispositif des revendications 13 à 45 à la stérilisation d'objets de toute forme et de toute nature, notamment métallique, composite ou thermosensible.

47. Application du dispositif des revendications 13 à 45 à la stérilisation des surfaces des emballages, des produits ou des matériels de production.

48. Application du dispositif des revendications 13 à 45 à la décontamination des surfaces internes des systèmes d'air conditionné

49. Application du dispositif des revendications 13 à 45 à la désinfection des zones de confinement ou de transfert.

## Claims

1. Process for the plasma sterilisation at a pressure equal or substantially equal to atmospheric pressure of at least one object, in which:
a) the object or objects (50) to be treated are placed in a treatment chamber (10) at substantially atmospheric pressure;
b) one or more non-biocidal gas mixtures, at least one of which contains moisture, are introduced into this treatment chamber;
c) a plasma, producing chemical species from one of the gas mixtures, is created by generating, by means of a high-voltage supply (38), an electrical discharge between a high-voltage electrode (36) and an earth electrode (40), these two electrodes being placed in this treatment chamber;
d) the chemical species of the plasma are carried away out of the inter-electrode region (30) to the surface of the object or objects (50) to be treated; and
e) the gas residues resulting from the treatment are removed from the treatment chamber.

2. Process according to Claim 1, **characterised in that** the moisture is introduced directly around the object or objects (50) to be treated.

3. Process according to Claim 1, **characterised in that** the moisture is introduced near the inter-electrode region (30).

4. Process according to any one of Claims 1 to 3, **characterised in that** the gas mixture contains at least 10% oxygen and 10% nitrogen.

5. Process according to Claim 4, **characterised in that** the gas mixture consists of ambient air.

6. Process according to any one of Claims 1 to 5, **characterised in that** the relative humidity around the object or objects (50) to be treated is between 50% and 100%.

7. Process according to Claim 6, **characterised in that** the relative humidity around the object or objects (50) to be treated is greater than or equal to 90%.

8. Process according to Claim 1, **characterised in that** step b) of introducing the gas mixture or mixtures into the treatment chamber (10) is carried out continuously or intermittently.

9. Process according to Claim 8, **characterised in that** the flow rate of the gas mixture or mixtures entering the treatment chamber (10) is controlled.

10. Process according to Claim 1, **characterised in that** step c) of creating the plasma is preceded by a step of forced circulation of the gas mixture or mixtures in the treatment chamber (10).

11. Process according to Claim 1, **characterised in that** step d) of carrying away the chemical species of the plasma to the surface (50) to be treated is accomplished by using the electrical wind created by the discharge between the two electrodes (36, 40).

12. Process according to Claim 1, **characterised in that** step d) of carrying away the chemical species of the plasma to the surface (50) to be treated is accomplished by creating a forced flow, in the treatment chamber (10).

13. Device for the plasma sterilisation of at least one object, **characterised in that** it comprises:
- a first gas source (12) containing a non-biocidal gas mixture
- at least one treatment chamber (10) at atmospheric pressure comprising at least one sterilisation region (10b, 32) in which the object or objects (50) to be treated are placed, this chamber furthermore including, in at least one plasma generation region (10a, 30) separate from the sterilisation region, at least two electrodes (36, 40) connected to a high-voltage supply (38) in order to create a plasma producing chemical species by generating an electrical discharge between these electrodes in the gas mixture introduced into the plasma generation region, the chemical species of the plasma being carried away out of the plasma generation region to the surface of the object or objects to be treated and, the gas residues resulting from the treatment, being removed to a recovery system (22) via an outlet port (44) of this chamber; and
- a humidifying chamber (14) connected downstream of a second gas source (12, 26) in order to maintain a defined moisture content around the object or objects (50) to be treated.

14. Device according to Claim 13, **characterised in that** the first and second gas sources form a single gas source (12).

15. Device according to Claim 14, **characterised in that** the plasma generation region (10a, 30) is connected to this single gas source via the humidifying chamber (14).

16. Device according to Claim 14, **characterised in that** the plasma generation region (10a, 30) is connected directly to this single gas source (12), the sterilisation region (10b, 32) being connected to this single gas source via the humidifying chamber (14).

17. Device according to Claim 13, **characterised in that** the sterilisation region (10b, 32) is connected to the second gas source (26) via the humidifying chamber (14), the plasma generation region (10a, 30) being connected directly to the first gas source (12).

18. Device according to Claim 16 or Claim 17, **characterised in that** it includes a second relative humidity sensor (18b) placed upstream of the sterilisation region (10b, 32).

19. Device according to any one of Claims 15 to 17, **characterised in that** it includes a first relative humidity sensor (18a) placed upstream of the plasma generation region (10a, 30).

20. Device according to any one of Claims 13 to 19, **characterised in that** the gas mixture contains at least 10% oxygen and 10% nitrogen.

21. Device according to Claim 20, **characterised in that** the gas mixture consists of ambient air.

22. Device according to Claim 21, **characterised in that** the ambient air is compressed before it is humidified.

23. Device according to any one of Claims 13 to 22, **characterised in that** the sterilisation region (10b, 32) has a relative humidity of between 50% and 100%, advantageously greater than or equal to 90%.

24. Device according to any one of Claims 13 to 23, **characterised in that** it comprises at least one electrode with a large radius of curvature and one electrode with a small radius of curvature, one being a high-voltage electrode (36) and the other being an earth electrode (40).

25. Device according to Claim 24, **characterised in that** the electrode with a small radius of curvature is a metal electrode which may have one of the following shapes : a wire, spikes or a wire having spikes.

26. Device according to Claim 24, **characterised in that** the electrode with a large radius of curvature is a metal electrode which may have one of the following shapes: a wire, a plan, or a mesh or solid cylinder.

27. Device according to Claim 25 or Claim 26, **characterised in that** one or the other of the two electrodes, or both electrodes, are covered with a dielectric coating.

28. Device according to Claim 25 and Claim 26, **characterised in that** the high-voltage electrode consists of a wire (162) and **in that** the earth electrode consists of a mesh cylinder (164) surrounding this wire.

29. Device according to Claim 24, **characterised in that** the electrodes are mounted as an array of parallel electrodes, the high-voltage electrodes being supplied in succession or simultaneously.

30. Device according to any one Claims 13 to 29, **characterised in that** the electrodes are of limited usage.

31. Device according to any one Claims 13 to 30, **characterised in that** the high-voltage supply (38) is provided by a low-frequency generator delivering a DC voltage, square-wave voltage or AC voltage, or a voltage pulsed over time.

32. Device according to Claim 13, **characterised in that** it comprises several treatment chambers, each treatment chamber having at least one plasma generation region (68; 70; 72; 132, 136, 138; 188) connected, fixedly or not, to at least one sterilisation region (76; 78; 80; 166; 206), the plasma generation regions being connected to a common central unit (60) containing at least the first non-biocidal gas source (12), the humidifying chamber (14), the gas residues recovery system (22) and the high-voltage supply (38) .

33. Device according to Claim 32, **characterised in that** the sterilisation region (166; 206) of the treatment chamber (74, 130) has a shape specially tailored to the object or objects (134, 140; 226, 228, 230) to be sterilised so as to limit the production of chemical species necessary for sterilisation and to optimise the rate of flow and the concentration of these sterilising species around the object.

34. Device according to Claim 33, **characterised in that** the treatment chamber (74) includes a case (186) of standard shape and containing the plasma generation region (188), the sterilisation region being formed by a removable support (196) especially tailored to the object or objects to be treated and housed in this case.

35. Device according to Claim 32 or Claim 34, **characterised in that** the sterilisation region includes propagation regions (200) of small cross section making it possible to accelerate the chemical species emanating from the plasma generation region (188) towards various parts of the object or objects to be treated.

36. Device according to Claim 32, **characterised in that** the plasma generation region is incorporated into the object to be treated and forms a part thereof.

37. Device according to Claim 32, **characterised in that** the sterilisation region is separate from the plasma generation region and forms an independent chamber (76, 78, 80).

38. Device according to Claim 32 or Claim 37, **characterised in that** one or more of the treatment or sterilisation chambers constitute reusable autonomous packaging, in the form of a transportation case, allowing the sterile post-treatment state to be maintained.

39. Device according to Claim 32 or Claim 37, **characterised in that** one or more of the treatment or sterilisation chambers constitute disposable packaging, in the form of a flexible bag (220), it being possible for the sterilisation region to be divided into several separate regions (234, 236, 238) after the treatment, by cutting and concomitantly sealing defined parts of this bag.

40. Device according to any one of Claims 32 to 39, **characterised in that** all or part of the device is placed in a Faraday cage.

41. Device according to Claim 32, **characterised in that** the common central unit (60) includes indicating and control means (84, 86, 88, 90) which are associated with each sterilisation chamber in order for the sterilisation of the objects that it contains to be controlled individually.

42. Device according to Claim 41, **characterised in that** the common central control unit (60) includes printing means (96) for printing a label (94) on which will be printed, for each sterilisation chamber connected to this central control unit, an identification number specific to each chamber together with the date of the treatment and the parameters of the stabilisation cycle carried out.

43. Device according to Claim 32, **characterised in that** the treatment or sterilisation chamber is provided with an electronic label (98a) which makes it possible, by means of a corresponding reader (98b) of the central control unit (60), to determine automatically the flow rate setpoint values and control current which are suitable for the object or objects to be treated and to calculate the time needed to sterilise these objects.

44. Device according to Claim 43, **characterised in that** the electronic label includes a velocity sensor for measuring the flow rate of the chemical species of the plasma in the chamber.

45. Device according to Claim 43, **characterised in that** the electronic label includes a chemical measurement sensor.

46. Application of the device of Claims 13 to 45 to the sterilisation of objects of any shape and of any nature, especially be they metallic, composite or heat-sensitive.

47. Application of the device of Claims 13 to 45 to the sterilisation of the surfaces of packaging, of products or of production equipment.

48. Application of the device of Claims 13 to 45 to the decontamination of the internal surfaces of air conditioning systems.

49. Application of the device of Claims 13 to 45 to the disinfection of containment or transfer areas.

## Patentansprüche

1. Verfahren zum Sterilisieren zumindest einen Gegenstands mittels Plasma mit einem Druck, der dem Atmosphärendruck gleich oder im Wesentlichen gleich ist, bei welchem:
a) man den zu behandelnden Gegenstand (50) oder die zu behandelnden Gegenstände in ein Behandlungsgehäuse (10) hinein verbringt, das im Wesentlichen Atmosphärendruck unterliegt,
b) man in dieses Behandlungsgehäuse eine oder mehrere nicht-biozidische Gasmischungen einbringt, von denen zumindest eine in sich Feuchtigkeit aufgenommen hat,
c) man ein Plasma erzeugt, wobei man ausgehend von einer der Gasmischungen chemische Produkte herstellt, indem man mittels einer Hochspannungsspeisung (38) zwischen einer Hochspannungselektrode (36) und einer Massenelektrode (40) eine elektrische Entladung hervorruft, wobei sich diese beiden Elektroden in diesem Behandlungsgehäuse befinden,
d) man die chemischen Produkte des Plasmas aus dem Zwischenelektrodenbereich (30) zur Oberfläche des zu behandelnden Gegenstands (50) oder der zu behandelnden Gegenstände leitet, und
e) man die Gasrückstände, die von der Behandlung herrühren, aus dem Behandlungsgehäuse abführt.

2. Verfahren nach Anspruch 1, **dadurch gekennzeichnet, dass** die Feuchtigkeit direkt bei dem zu behandelnden Gegenstand (50) oder den zu behandelnden Gegenständen eingebracht wird.

3. Verfahren nach Anspruch 1, **dadurch gekennzeichnet, dass** die Feuchtigkeit beim Zwischenelektrodenbereich (30) eingebracht wird.

4. Verfahren nach einem der Ansprüche 1 bis 3, **dadurch gekennzeichnet, dass** die Gasmischung zumindest 10 % Sauerstoff und 10 % Stickstoff enthält.

5. Verfahren nach Anspruch 4, **dadurch gekennzeichnet, dass** die Gasmischung in Form von Außenluft ist.

6. Verfahren nach einem der Ansprüche 1 bis 5, **dadurch gekennzeichnet, dass** der relative Feuchtigkeitsgrad bei dem zu behandelnden Gegenstand (50) oder den zu behandelnden Gegenständen zwischen 50 % und 100 % liegt.

7. Verfahren nach Anspruch 6, **dadurch gekennzeichnet, dass** der relative Feuchtigkeitsgrad bei dem zu behandelnden Gegenstand (50) oder den zu behandelnden Gegenständen (50) größer oder gleich 90 % ist.

8. Verfahren nach Anspruch 1, **dadurch gekennzeichnet, dass** der Schritt b) des Einbringens der Gasmischung(en) in das Behandlungsgehäuse (10) in kontinuierlicher oder intermittierender Weise erfolgt.

9. Verfahren nach Anspruch 8, **dadurch gekennzeichnet, dass** eine Steuerung des Durchsatzes der in das Behandlungsgehäuse (10) eindringenden Gasmischung(en) vorgenommen wird.

10. Verfahren nach Anspruch 1, **dadurch gekennzeichnet, dass** dem Schritt c) der Erzeugung des Plasmas ein Schritt des Bewirkens einer Umwälzung der Gasmischung(en) im Behandlungsgehäuse (10) vorangeht.

11. Verfahren nach Anspruch 1, **dadurch gekennzeichnet, dass** der Schritt d) des Leitens der chemischen Produkte des Plasmas zur zu behandelnden Oberfläche (50) mittels der Verwendung von elektrischem Wind, der durch die Entladung zwischen den beiden Elektroden (36, 40) erzeugt wird, erfolgt.

12. Verfahren nach Anspruch 1, **dadurch gekennzeichnet, dass** der Schritt d) des Leitens der chemischen Produkte des Plasmas zur zu behandelnden Oberfläche (50) mittels der Erzeugung einer erzwungenen Strömung in dem Behandlungsgehäuse (10) erfolgt.

13. Vorrichtung zum Sterilisieren zumindest eines Gegenstands mittels Plasma, **dadurch gekennzeichnet, dass** sie umfasst:
- eine erste Gasquelle (12), welche eine nicht-biozidische Gasmischung enthält,
- zumindest ein Behandlungsgehäuse (10), welches Atmosphärendruck unterworfen ist und zumindest einen Bereich zum Sterilisieren (10b, 32) umfasst, in welchem sich der zu behandelnde Gegenstand (50) oder die zu behandelnden Gegenstände befinden, wobei dieses Gehäuse ferner zumindest einen Bereich (10a, 30) zum Erzeugen von Plasma umfasst, der von dem Bereich zum Sterilisieren verschieden ist, wobei zumindest zwei Elektroden (36, 40) mit einer Hochspannungsspeisung (38) verbunden sind, um ein Plasma zu erzeugen, welches mittels der Erzeugung einer elektrischen Entladung zwischen diesen Elektroden ausgehend von der in dem Bereich zum Erzeugen von Plasma eingebrachten Gasmischung chemische Produkte herstellt, wobei die chemischen Produkte des Plasmas aus dem Bereich zum Erzeugen des Plasmas zur Oberfläche des zu behandelnden Gegenstands oder der zu behandelnden Gegenstände geleitet werden, und wobei die von der Behandlung herrührenden Gasrückstände über eine Ausgangsöffnung (44) dieses Gehäuses zu einem Sammelsystem (22) hin abgeführt werden, und
- eine Befeuchtungskammer (14), die mit dem Ausgang einer zweiten Gasquelle (12, 26) verbunden ist, um bei dem zu behandelnden Gegenstand (50) oder den zu behandelnden Gegenständen einen bestimmten Feuchtigkeitsgrad aufrechtzuerhalten.

14. Vorrichtung nach Anspruch 13, **dadurch gekennzeichnet, dass** die erste und zweite Gasquelle eine einzige Gasquelle (12) bilden.

15. Vorrichtung nach Anspruch 14, **dadurch gekennzeichnet, dass** der Bereich zum Erzeugen von Plasma (10a, 30) über die Befeuchtungskammer (14) mit dieser einzigen Gasquelle verbunden ist.

16. Vorrichtung nach Anspruch 14, **dadurch gekennzeichnet, dass** der Bereich zum Erzeugen von Plasma (10a, 30) mit dieser einzigen Gasquelle (12) direkt verbunden ist, wobei der Bereich zum Sterilisieren (10b, 32) über die Befeuchtungskammer (14) mit dieser einzigen Gasquelle verbunden ist.

17. Vorrichtung nach Anspruch 13, **dadurch gekennzeichnet, dass** der Bereich zum Sterilisieren (10b, 32) über die Befeuchtungskammer (14) mit der zweiten Gasquelle (26) verbunden ist, wobei der Bereich zum Erzeugen von Plasma (10a, 30) direkt mit der ersten Gasquelle (12) verbunden ist.

18. Vorrichtung nach Anspruch 16 oder Anspruch 17, **dadurch gekennzeichnet, dass** sie zumindest einen zweiten Sensor (18b) für die relative Feuchtigkeit umfasst, der sich am Eingang des Bereichs zum Sterilisieren (10b, 32) befindet.

19. Vorrichtung nach einem der Ansprüche 15 bis 17, **dadurch gekennzeichnet, dass** sie einen ersten Sensor für relative Feuchtigkeit (18a) umfasst, der sich am Eingang des Bereichs zum Erzeugen von Plasma (10a, 30) befindet.

20. Vorrichtung nach einem der Ansprüche 13 bis 19, **dadurch gekennzeichnet, dass** die Gasmischung zumindest 10 % Sauerstoff und 10 % Stickstoff enthält.

21. Vorrichtung nach Anspruch 20, **dadurch gekennzeichnet, dass** die Gasmischung in Form von Außenluft ist.

22. Vorrichtung nach Anspruch 21, **dadurch gekennzeichnet, dass** die Außenluft vor ihrer Befeuchtung komprimiert wird.

23. Vorrichtung nach einem der Ansprüche 13 bis 22, **dadurch gekennzeichnet, dass** im Bereich zum Sterilisieren (10b, 32) ein relativer Feuchtigkeitsgrad herrscht, der zwischen 50 % und 100 % liegt und vorteilhafterweise größer oder gleich 90 % ist.

24. Vorrichtung nach einem der Ansprüch13 bis 23, **dadurch gekennzeichnet, dass** er zumindest eine Elektrode mit großem Krümmungsradius und eine Elektrode mit kleinem Krümmungsradius umfasst, wobei die eine eine Hochspannungselektrode (36) und die andere eine Massenelektrode (40) ist.

25. Vorrichtung nach Anspruch 24, **dadurch gekennzeichnet, dass** die Elektrode mit kleinem Krümmungsradius eine metallische Elektrode ist, die eine der folgenden Formen einnehmen kann: Draht, Spitzen oder Draht mit Spitzen.

26. Vorrichtung nach Anspruch 24, **dadurch gekennzeichnet, dass** die Elektrode mit großem Krümmungsradius eine metallische Elektrode ist, die eine der folgenden Formen einnehmen kann: Draht, Ebene, Vollzylinder oder Gitter.

27. Vorrichtung nach Anspruch 25 oder Anspruch 26, **dadurch gekennzeichnet, dass** die eine oder die andere der beiden Elektroden oder beide mit einer dielektrischen Beschichtung überzogen ist/sind.

28. Vorrichtung nach Anspruch 25 und Anspruch 26, **dadurch gekennzeichnet, dass** die Hochspannungselektrode von einem Draht (162) gebildet ist, und **dass** die Massenelektrode von einem den Draht umgebenden Gitterzylinder (164) gebildet ist.

29. Vorrichtung nach Anspruch 24, **dadurch gekennzeichnet, dass** die Elektroden als Netz paralleler Elektroden installiert sind, wobei die Speisung der Elektroden mit Hochspannung aufeinanderfolgend oder simultan erfolgt.

30. Vorrichtung nach einem der Ansprüche 13 bis 29, **dadurch gekennzeichnet, dass** die Elektroden Verbrauchselektroden sind.

31. Vorrichtung nach einem der Ansprüche 13 bis 30, **dadurch gekennzeichnet, dass** die Hochspannungsspeisung (38) mittels eines Niederfrequenzgenerators bereitgestellt wird, der eine Gleichspannung, eine Rechteckspannung, alternativ oder in zeitlichen Pulsen, abgibt.

32. Vorrichtung nach Anspruch 13, **dadurch gekennzeichnet, dass** sie mehrere Behandlungsgehäuse umfasst, wobei jedes Behandlungsgehäuse zumindest einen Bereich zum Erzeugen von Plasma (68; 70; 72; 132, 136, 138; 188), der fest oder auch nicht mit zumindest einem Bereich zum Sterilisieren (76; 78; 80; 166; 206) verbunden ist, wobei die Bereiche zum Erzeugen von Plasma mit einer gemeinsamen zentralen Einheit (60) verbunden sind, welche zumindest die erste Quelle (12) von nicht-biozidischem Gas, die Befeuchtungskammer (14), das System (22) zum Sammeln von Gasrückständen und die Hochspannungsspeisung (38) umfasst.

33. Vorrichtung nach Anspruch 32, **dadurch gekennzeichnet, dass** der Bereich zum Sterilisieren (166; 206) des Behandlungsgehäuses (74, 130) eine Form aufweist, die besonders an den zu sterilisierenden Gegenstand oder die zu sterilisierenden Gegenstände (134, 140; 226, 228, 230) angepasst ist, um die Erzeugung von chemischen Produkten, welche zum Sterilisieren erforderlich sind, einzuschränken, und um die Strömungsgeschwindigkeit und die Konzentration dieser sterilisierenden Produkte um den Gegenstand herum zu optimieren.

34. Vorrichtung nach Anspruch 33, **dadurch gekennzeichnet, dass** das Behandlungsgehäuse (74) eine Box (186) mit Standardform, welche den Bereich zum Erzeugen von Plasma (188) enthält, umfasst, wobei der Bereich zum Sterilisieren von einem entnehmbaren Träger (196) gebildet ist, der besonders an den zu behandelnden Gegenstand oder die zu behandelnden Gegenstände angepasst ist und in die Box eingebettet ist.

35. Vorrichtung nach Anspruch 32 oder 34, **dadurch gekennzeichnet, dass** der Bereich zum Sterilisieren Ausbreitungsbereiche (200) mit kleinem Querschnitt umfasst, die es erlauben, die von dem Bereich zum Erzeugen von Plasma (188) herkommenden chemischen Produkte zu verschiedenen Teilen des zu behandelnden Gegenstands oder der zu behandelnden Gegenstände hin zu beschleunigen.

36. Vorrichtung nach Anspruch 32, **dadurch gekennzeichnet, dass** der Bereich zum Erzeugen von Plasma in den zu behandelnden Gegenstand integriert ist, von dem er einen Teil bildet.

37. Vorrichtung nach Anspruch 32, **dadurch gekennzeichnet, dass** der Bereich zum Sterilisieren von dem Bereich zum Erzeugen von Plasma getrennt ist und ein unabhängiges Gehäuse (76, 78, 80) bildet.

38. Vorrichtung nach Anspruch 32 oder Anspruch 37, **dadurch gekennzeichnet, dass** ein oder mehrere Gehäuse zum Behandeln oder zum Sterilisieren eine selbstständige wiederverwertbare Verpackung in Form eines Transportköfferchens bildet, welches es erlaubt, nach der Behandlung den sterilen Zustand zu bewahren.

39. Vorrichtung nach Anspruch 32 oder Anspruch 37, **dadurch gekennzeichnet, dass** ein oder mehrere Gehäuse zum Behandeln oder zum Sterilisieren eine Wegwerfpackung in Form eines weichen Beutels (220) bilden, wobei der Bereich zum Sterilisieren durch Zerschneiden und gleichzeitiges Versiegeln von bestimmten Teilen dieses Beutels in mehrere, nach dem Behandeln abgesonderte Bereiche (234, 236, 238) getrennt werden kann.

40. Vorrichtung nach einem der Ansprüche 32 bis 39, **dadurch gekennzeichnet, dass** sich die ganze Vorrichtung oder ein Teil von ihr in einem Faradaykäfig befindet.

41. Vorrichtung nach Anspruch 32, **dadurch gekennzeichnet, dass** die gemeinsame zentrale Einheit (60) Mittel zum Anzeigen und zum Steuern (84, 86, 88, 90) umfasst, die jedem Sterilisierungsgehäuse zugeordnet sind, um eine individuelle Steuerung der Sterilisierung der Gegenstände, die es enthält, zu gewährleisten.

42. Vorrichtung nach Anspruch 41, **dadurch gekennzeichnet, dass** die gemeinsame zentrale Einheit (60) Mittel zum Drucken (96) umfasst, um ein Etikett (94) zu drucken, auf dem für jedes mit dieser zentralen Einheit verbundene Sterilisierungsgehäuse eine Identifizierungsnummer, die für jedes Gehäuse spezifisch ist, wie auch das Datum der Behandlung und die Parameter des durchgeführten Sterilisierungszyklus aufgedruckt sind.

43. Vorrichtung nach Anspruch 32, **dadurch gekennzeichnet, dass** das Gehäuse zum Behandeln oder zum Sterilisieren mit einem elektronischen Etikett (98a) versehen ist, welches es über einen entsprechenden Leser (98b) der zentralen Einheit (60) erlaubt, die Werte der Zuflussströmung und des Regulierungsstroms, die für den zu behandelnden Gegenstand oder die zu behandelnden Gegenstände geeignet sind, automatisch zu bestimmen, und die für die Sterilisierung dieser Gegenstände erforderliche Zeit zu berechnen.

44. Vorrichtung nach Anspruch 43, **dadurch gekennzeichnet, dass** das elektronische Etikett einen Geschwindigkeitssensor umfasst, um die Geschwindigkeit der Strömung der chemischen Produkte des Plasmas in dem Gehäuse zu messen.

45. Vorrichtung nach Anspruch 43, **dadurch gekennzeichnet, dass** das elektronische Etikett einen Sensor für eine chemische Messung umfasst.

46. Verwendung der Vorrichtung nach den Ansprüchen 13 bis 45 zum Sterilisieren von Gegenständen jeglicher Form und jeglicher Art, insbesondere metallischer, zusammengesetzter oder thermosensibler Art.

47. Verwendung der Vorrichtung nach den Ansprüchen 13 bis 45 zum Sterilisieren von Oberflächen von Verpackungen, Produkten oder Produktionsmitteln.

48. Verwendung der Vorrichtung nach den Ansprüchen 13 bis 45 zum Dekontaminieren von inneren Oberflächen von Klimaanlagen.

49. Verwendung der Vorrichtung nach den Ansprüchen 13 bis 45 zum Desinfizieren betreffend Umschließungsbereiche oder Überführungsbereiche.
